# EUROPEAN PATENT SPECIFICATION

(11) **EP 2 914 619 B1**
(45) Date of publication and mention of the grant of the patent: **29.05.2019**
(21) Application number: 13851277.7
(22) Date of filing: 31.10.2013
(51) Int. Cl.: C07K 14/435, C07K 7/08, C12N 15/12, C07K 16/18

(54) **ERYTHROFERRONE AND ERFE POLYPEPTIDES AND METHODS OF REGULATING IRON METABOLISM**
ERYTHROFERRON- UND ERFE-POLYPEPTIDE SOWIE VERFAHREN ZUR REGULIERUNG DES EISENSTOFFWECHSELS
POLYPEPTIDES ÉRYTHROFERRONE ET ERFE, ET PROCÉDÉS DE RÉGULATION DU MÉTABOLISME DU FER

(30) Priority: 01.11.2012 US 201261721322 P
(43) Date of publication of application: 09.09.2015
(73) Proprietor: The Regents of the University of California, Oakland, CA 94607 (US)
(72) Inventor: GANZ, Tomas, Los Angeles, California 90095-1690 (US); NEMETH, Elizabeta, Los Angeles, California 90095-1690 (US); KAUTZ, Leon, Los Angeles, California 90095-1690 (US)
(74) Representative: HGF Limited
(86) International application number: PCT/US2013/067771
(87) International publication number: WO 2014/071015

(56) References cited:
- WO-A1-2010/040998
- WO-A1-2013/112663
- WO-A2-2006/046073
- US-A1- 2008 213 277
- US-A1- 2012 040 894
- M. M. SELDIN ET AL: "Myonectin (CTRP15), a Novel Myokine That Links Skeletal Muscle to Systemic Lipid Homeostasis", JOURNAL OF BIOLOGICAL CHEMISTRY, vol. 287, no. 15, 17 February 2012 (2012-02-17), pages 11968-11980, XP055158331, ISSN: 0021-9258, DOI: 10.1074/jbc.M111.336834
- LÉON KAUTZ ET AL: "Identification of erythroferrone as an erythroid regulator of iron metabolism", NATURE GENETICS., vol. 46, no. 7, 1 June 2014 (2014-06-01), pages 678-684, XP55259858, NEW YORK, US ISSN: 1061-4036, DOI: 10.1038/ng.2996
- DATABASE GENBANK [Online] 17 July 2012 Database accession no. XP_002813099.1
- HUNTER, HOWARD N. ET AL.: 'The solution structure of human hepcidin, a peptide hormone with antimicrobial activity that is involved in iron uptake and hereditary hemochromatosis' THE JOURNAL OF BIOLOGICAL CHEMISTRY vol. 277, no. 40, 04 October 2002, pages 37597 - 37603, XP002519930

### SEQUENCE LISTING

<110> The Regents of the University of California
<120> ERYTHROFERRONE AND ERFE POLYPEPTIDES AND METHODS OF REGULATING IRON METABOLISM
<130> 034044.119WO1
<150> 61/721,322
   <151> 2012-11-01
<160> 18
<170> PatentIn version 3.5
<210> 1
   <211> 354
   <212> PRT
   <213> Homo sapiens
<400> 1
   Met Ala Pro Ala Arg Arg Pro Ala Gly Ala Arg Leu Leu Leu Val Tyr
   1               5                   10                  15
   Ala Gly Leu Leu Ala Ala Ala Ala Ala Gly Leu Gly Ser Pro Glu Pro
               20                  25                  30
   Gly Ala Pro Ser Arg Ser Arg Ala Arg Arg Glu Pro Pro Pro Gly Asn
           35                  40                  45
   Glu Leu Pro Arg Gly Pro Gly Glu Ser Arg Ala Gly Pro Ala Ala Arg
       50                  55                  60
   Pro Pro Glu Pro Thr Ala Glu Arg Ala His Ser Val Asp Pro Arg Asp
   65                  70                  75                  80
   Ala Trp Met Leu Phe Val Arg Gln Ser Asp Lys Gly Val Asn Gly Lys
                   85                  90                  95
   Lys Arg Ser Arg Gly Lys Ala Lys Lys Leu Lys Phe Gly Leu Pro Gly
               100                 105                 110
   Pro Pro Gly Pro Pro Gly Pro Gln Gly Pro Pro Gly Pro Ile Ile Pro
           115                 120                 125
   Pro Glu Ala Leu Leu Lys Glu Phe Gln Leu Leu Leu Lys Gly Ala Val
       130                 135                 140
   Arg Gln Arg Glu Arg Ala Glu Pro Glu Pro Cys Thr Cys Gly Pro Ala
   145                 150                 155                 160
   Gly Pro Val Ala Ala Ser Leu Ala Pro Val Ser Ala Thr Ala Gly Glu
   165 170 175
   Asp Asp Asp Asp Val Val Gly Asp Val Leu Ala Leu Leu Ala Ala Pro
   180 185 190
   Leu Ala Pro Gly Pro Arg Ala Pro Arg Val Glu Ala Ala Phe Leu Cys
   195 200 205
   Arg Leu Arg Arg Asp Ala Leu Val Glu Arg Arg Ala Leu His Glu Leu
   210 215 220
   Gly Val Tyr Tyr Leu Pro Asp Ala Glu Gly Ala Phe Arg Arg Gly Pro
   225 230 235 240
   Gly Leu Asn Leu Thr Ser Gly Gln Tyr Arg Ala Pro Val Ala Gly Phe
   245 250 255
   Tyr Ala Leu Ala Ala Thr Leu His Val Ala Leu Gly Glu Pro Pro Arg
   260 265 270
   Arg Gly Pro Pro Arg Pro Arg Asp His Leu Arg Leu Leu Ile Cys Ile
   275 280 285
   Gln Ser Arg Cys Gln Arg Asn Ala Ser Leu Glu Ala Ile Met Gly Leu
   290 295 300
   Glu Ser Ser Ser Glu Leu Phe Thr Ile Ser Val Asn Gly Val Leu Tyr
   305 310 315 320
   Leu Gln Met Gly Gln Trp Thr Ser Val Phe Leu Asp Asn Ala Ser Gly
   325 330 335
   Cys Ser Leu Thr Val Arg Ser Gly Ser His Phe Ser Ala Val Leu Leu
   340 345 350
   Gly Val
<210> 2
   <211> 340
   <212> PRT
   <213> Mus musculus
<400> 2
   Met Ala Ser Thr Arg Arg Pro Val Gly Ala Arg Thr Leu Leu Ala Cys
   1 5 10 15
   Ala Ser Leu Leu Ala Ala Met Gly Leu Gly Val Pro Glu Ser Ala Glu
               20                  25                  30
   Pro Val Gly Thr His Ala Arg Pro Gln Pro Pro Gly Ala Glu Leu Pro
           35                  40                  45
   Ala Pro Pro Ala Asn Ser Pro Pro Glu Pro Thr Ile Ala His Ala His
       50                  55                  60
   Ser Val Asp Pro Arg Asp Ala Trp Met Leu Phe Val Lys Gln Ser Asp
   65                  70                  75                  80
   Lys Gly Ile Asn Ser Lys Arg Arg Ser Lys Ala Arg Arg Leu Lys Leu
                   85                  90                  95
   Gly Leu Pro Gly Pro Pro Gly Pro Pro Gly Pro Gln Gly Pro Pro Gly
               100                 105                 110
   Pro Phe Ile Pro Ser Glu Val Leu Leu Lys Glu Phe Gln Leu Leu Leu
           115                 120                 125
   Lys Gly Ala Val Arg Gln Arg Glu Ser His Leu Glu His Cys Thr Arg
       130                 135                 140
   Asp Leu Thr Thr Pro Ala Ser Gly Ser Pro Ser Arg Val Pro Ala Ala
   145                 150                 155                 160
   Gln Glu Leu Asp Ser Gln Asp Pro Gly Ala Leu Leu Ala Leu Leu Ala
                   165                 170                 175
   Ala Thr Leu Ala Gln Gly Pro Arg Ala Pro Arg Val Glu Ala Ala Phe
               180                 185                 190
   His Cys Arg Leu Arg Arg Asp Val Gln Val Asp Arg Arg Ala Leu His
           195                 200                 205
   Glu Leu Gly Ile Tyr Tyr Leu Pro Glu Val Glu Gly Ala Phe His Arg
       210                 215                 220
   Gly Pro Gly Leu Asn Leu Thr Ser Gly Gln Tyr Thr Ala Pro Val Ala
   225                 230                 235                 240
   Gly Phe Tyr Ala Leu Ala Ala Thr Leu His Val Ala Leu Thr Glu Gln
                   245                 250                 255
   Pro Arg Lys Gly Pro Thr Arg Pro Arg Asp Arg Leu Arg Leu Leu Ile
               260                 265                 270
   Cys Ile Gln Ser Leu Cys Gln His Asn Ala Ser Leu Glu Thr Val Met
           275                 280                 285
   Gly Leu Glu Asn Ser Ser Glu Leu Phe Thr Ile Ser Val Asn Gly Val
       290                 295                 300
   Leu Tyr Leu Gln Ala Gly His Tyr Thr Ser Val Phe Leu Asp Asn Ala
   305                 310                 315                 320
   Ser Gly Ser Ser Leu Thr Val Arg Ser Gly Ser His Phe Ser Ala Ile
                   325                 330                 335
   Leu Leu Gly Leu
               340
<210> 3
   <211> 17
   <212> PRT
   <213> Artificial Sequence
<220>
   <223> Synthetic peptide based on sequences from Homo sapiens and Mus musculus.
<400> 3
   Gly Leu Pro Gly Pro Pro Gly Pro Pro Gly Pro Gln Gly Pro Pro Gly
   1               5                   10                  15
   Pro
<210> 4
   <211> 14
   <212> PRT
   <213> Artificial Sequence
<220>
   <223> Synthetic peptide based on sequences from Homo sapiens and Mus musculus.
<400> 4
   Ala His Ser Val Asp Pro Arg Asp Ala Trp Met Leu Phe Val
   1               5                   10
<210> 5
   <211> 22
   <212> PRT
   <213> Artificial Sequence
<220>
   <223> Synthetic peptide based on sequences from Homo sapiens and Mus musculus.
<220>
   <221> misc_feature
   <222> (15)..(15)
   <223> Xaa can be any naturally occurring amino acid
<220>
   <221> misc_feature
   <222> (21)..(21)
   <223> Xaa can be any naturally occurring amino acid
<400> 5
   Ala His Ser Val Asp Pro Arg Asp Ala Trp Met Leu Phe Val Xaa Gln
   1               5                   10                  15
   Ser Asp Lys Gly Xaa Asn
               20
<210> 6
   <211> 17
   <212> PRT
   <213> Artificial Sequence
<220>
   <223> Synthetic peptide based on sequences from Homo sapiens and Mus musculus.
<400> 6
   Leu Leu Lys Glu Phe Gln Leu Leu Leu Lys Gly Ala Val Arg Gln Arg
   1               5                   10                  15
   Glu
<210> 7
   <211> 11
   <212> PRT
   <213> Artificial Sequence
<220>
   <223> Synthetic peptide based on sequences from Homo sapiens and Mus musculus.
<400> 7
   Gly Pro Arg Ala Pro Arg Val Glu Ala Ala Phe
   1               5                   10
<210> 8
   <211> 16
   <212> PRT
   <213> Artificial Sequence
<220>
   <223> Synthetic peptide based on sequences from Homo sapiens and Mus musculus.
<220>
   <221> misc_feature
   <222> (2)..(2)
   <223> Xaa can be any naturally occurring amino acid
<220>
   <221> misc_feature
   <222> (11)..(11)
   <223> Xaa can be any naturally occurring amino acid
<220>
   <221> misc_feature
   <222> (16)..(16)
   <223> Xaa can be any naturally occurring amino acid
<400> 8
   Val Xaa Arg Arg Ala Leu His Glu Leu Gly Xaa Tyr Tyr Leu Pro Xaa
   1               5                   10                  15
<210> 9
   <211> 9
   <212> PRT
   <213> Artificial Sequence
<220>
   <223> Synthetic peptide based on sequences from Homo sapiens and Mus musculus.
<400> 9
   Gly Leu Asn Leu Thr Ser Gly Gln Tyr
   1               5
<210> 10
   <211> 17
   <212> PRT
   <213> Artificial Sequence
<220>
   <223> Synthetic peptide based on sequences from Homo sapiens and Mus musculus.
<400> 10
   Ala Pro Val Ala Gly Phe Tyr Ala Leu Ala Ala Thr Leu His Val Ala
   1               5                   10                  15
   Leu
<210> 11
   <211> 22
   <212> PRT
   <213> Artificial Sequence
<220>
   <223> Synthetic peptide based on sequences from Homo sapiens and Mus musculus.
<220>
   <221> misc_feature
   <222> (1)..(1)
   <223> Xaa can be any naturally occurring amino acid
<220>
   <221> misc_feature
   <222> (6)..(6)
   <223> Xaa can be any naturally occurring amino acid
<400> 11
   Xaa Met Gly Leu Glu Xaa Ser Ser Glu Leu Phe Thr Ile Ser Val Asn
   1               5                   10                  15
   Gly Val Leu Tyr Leu Gln
               20
<210> 12
   <211> 16
   <212> PRT
   <213> Artificial Sequence
<220>
   <223> Synthetic peptide based on sequences from Homo sapiens and Mus musculus.
<400> 12
   Ser Ser Glu Leu Phe Thr Ile Ser Val Asn Gly Val Leu Tyr Leu Gln
   1               5                   10                  15
<210> 13
   <211> 10
   <212> PRT
   <213> Artificial Sequence
<220>
   <223> Synthetic peptide based on sequences from Homo sapiens and Mus musculus.
<400> 13
   Thr Ser Val Phe Leu Asp Asn Ala Ser Gly
   1               5                   10
<210> 14
   <211> 12
   <212> PRT
   <213> Artificial Sequence
<220>
   <223> Synthetic peptide based on sequences from Homo sapiens and Mus musculus.
<400> 14
   Ser Leu Thr Val Arg Ser Gly Ser His Phe Ser Ala
   1               5                   10
<210> 15
   <211> 17
   <212> PRT
   <213> Artificial Sequence
<220>
   <223> Synthetic peptide based on sequences from Homo sapiens and Mus musculus.
<220>
   <221> misc_feature
   <222> (13)..(13)
   <223> Xaa can be any naturally occurring amino acid
<220>
   <221> misc_feature
   <222> (17)..(17)
   <223> Xaa can be any naturally occurring amino acid
<400> 15
   Ser Leu Thr Val Arg Ser Gly Ser His Phe Ser Ala Xaa Leu Leu Gly
   1 5 10 15
   Xaa
<210> 16
   <211> 220
   <212> PRT
   <213> Homo sapiens
<400> 16
   Glu Phe Gln Leu Leu Leu Lys Gly Ala Val Arg Gln Arg Glu Arg Ala
   1 5 10 15
   Glu Pro Glu Pro Cys Thr Cys Gly Pro Ala Gly Pro Val Ala Ala Ser
   20 25 30
   Leu Ala Pro Val Ser Ala Thr Ala Gly Glu Asp Asp Asp Asp Val Val
   35 40 45
   Gly Asp Val Leu Ala Leu Leu Ala Ala Pro Leu Ala Pro Gly Pro Arg
   50 55 60
   Ala Pro Arg Val Glu Ala Ala Phe Leu Cys Arg Leu Arg Arg Asp Ala
   65 70 75 80
   Leu Val Glu Arg Arg Ala Leu His Glu Leu Gly Val Tyr Tyr Leu Pro
   85 90 95
   Asp Ala Glu Gly Ala Phe Arg Arg Gly Pro Gly Leu Asn Leu Thr Ser
   100 105 110
   Gly Gln Tyr Arg Ala Pro Val Ala Gly Phe Tyr Ala Leu Ala Ala Thr
   115 120 125
   Leu His Val Ala Leu Gly Glu Pro Pro Arg Arg Gly Pro Pro Arg Pro
   130 135 140
   Arg Asp His Leu Arg Leu Leu Ile Cys Ile Gln Ser Arg Cys Gln Arg
   145 150 155 160
   Asn Ala Ser Leu Glu Ala Ile Met Gly Leu Glu Ser Ser Ser Glu Leu
   165 170 175
   Phe Thr Ile Ser Val Asn Gly Val Leu Tyr Leu Gln Met Gly Gln Trp
   180 185 190
   Thr Ser Val Phe Leu Asp Asn Ala Ser Gly Cys Ser Leu Thr Val Arg
   195 200 205
   Ser Gly Ser His Phe Ser Ala Val Leu Leu Gly Val
   210 215 220
<210> 17
   <211> 134
   <212> PRT
   <213> Homo sapiens
<400> 17
   Met Ala Pro Ala Arg Arg Pro Ala Gly Ala Arg Leu Leu Leu Val Tyr
   1 5 10 15
   Ala Gly Leu Leu Ala Ala Ala Ala Ala Gly Leu Gly Ser Pro Glu Pro
   20 25 30
   Gly Ala Pro Ser Arg Ser Arg Ala Arg Arg Glu Pro Pro Pro Gly Asn
   35 40 45
   Glu Leu Pro Arg Gly Pro Gly Glu Ser Arg Ala Gly Pro Ala Ala Arg
   50 55 60
   Pro Pro Glu Pro Thr Ala Glu Arg Ala His Ser Val Asp Pro Arg Asp
   65 70 75 80
   Ala Trp Met Leu Phe Val Arg Gln Ser Asp Lys Gly Val Asn Gly Lys
                   85                  90                  95
   Lys Arg Ser Arg Gly Lys Ala Lys Lys Leu Lys Phe Gly Leu Pro Gly
               100                 105                 110
   Pro Pro Gly Pro Pro Gly Pro Gln Gly Pro Pro Gly Pro Ile Ile Pro
           115                 120                 125
   Pro Glu Ala Leu Leu Lys
       130
<210> 18
   <211> 10
   <212> PRT
   <213> Artificial Sequence
<220>
   <223> Synthetic peptide based on sequences from Homo sapiens and Mus musculus.
<400> 18
   Pro Ser Arg Val Pro Ala Ala Gln Glu Leu
   1               5                   10

## Description

### REFERENCE TO A SEQUENCE LISTING SUBMITTED VIA EFS-WEB

The content of the ASCII text file of the sequence listing named "20131031_034044_119WO1_seq_ST25" which is 14.3 kb in size was created on 28 October 2013, and electronically submitted via EFS-Web herewith the application.

### ACKNOWLEDGEMENT OF GOVERNMENT SUPPORT

This invention was made with Government support of Grant Nos. R01 DK 082717 and RO1 DK 065029, awarded by The National Institute of Diabetes and Digestive and Kidney Diseases (NIDDK) of the National Institutes of Health. The Government has certain rights in this invention.

### BACKGROUND OF THE INVENTION

WO 2006/046073 describes the protein INSP162, which has identity to SEQ ID NO: 1 disclosed in the present disclosure.

WO 2010/040998 describes a peptide SEQ ID NO: 37 which has sequence similarity to SEQ ID NO: 3 disclosed in the present disclosure.

### FIELD OF THE DISCLOSURE

The present disclosure relates to polypeptides which regulate iron metabolism and methods of making and using thereof.

### 2. DESCRIPTION OF THE RELATED ART.

Red cell production is by far the main consumer of iron in the body. The existence of hormones that regulate iron in response to the needs of red cell production has been proposed more than 50 years ago by Finch et al. among others. See Finch 1994. Unfortunately, Finch and other never discovered any such hormones.

Other investigators examined the causes of increased iron absorption in thalassemia and proposed that two substances, GDF15 and TWSG1, may cause hepcidin suppression and iron overload in this disease, but concluded that this was not the physiological signal for iron absorption. See Tanno 2010b and Casanovas 2013. The role of GDF15 or TWSG1 in the iron overload of the thalassemia was never specifically shown.

### SUMMARY OF THE INVENTION

The invention is directed to a monoclonal antibody which specifically recognizes an ERFE polypeptide or a composition thereof for use in treating an iron overload disease and/or disorder associated with abnormally low levels of hepcidin, wherein the ERFE polypeptide comprises an amino acid sequence selected from at least one of SEQ ID Nos: 1, 3, 4, 6, 7, 9, 10, 12-14, AHSVDPRDAWMLFVX₁QSDKGX₂N (SEQ ID NO:5) wherein X₁ is R and X₂, is V; VX₁RRALHELGX₂YYLPX₃ (SEQ ID NO:8) wherein X₁ is E, X₂, is V and X₃, is D; X₁MGLE X₂SSELFTISVNGVLYLQ (SEQ ID NO:11) wherein X₁ is I and X₂, is S; and SLTVRSGSHFSAX₁LLGX₂ (SEQ ID NO:15) wherein X₁ is V and X₂, is V, wherein the iron overload disease and/or disorder associated with abnormally low levels of hepcidin is selected from the group consisting of: myelodysplastic syndromes, hereditary hemochromatosis, iron-loading anemias, alcoholic liver diseases, chronic hepatitis C, alpha-thalassemia, beta-thalassemia, congenital dyserythropoietic anemias, and chronic hepatitis B.

The monoclonal antibody or a composition thereof for the use of the invention may comprise a pharmaceutically acceptable carrier or diluent.

In some embodiments, the monoclonal antibody is raised against a polypeptide comprising an amino acid sequence of SEQ ID NO: 1.

The present disclosure is directed to an isolated, purified, synthetic, and/or recombinant polypeptide which comprises a C-terminal sequence having about 95-100%, at least about 96%, at least about 97%, at least about 98%, or at least about 99% sequence identity to SEQ ID NO:16. In some embodiments, the polypeptide comprises an N-terminal sequence having about 70-100%, at least about 80%, at least about 90%, or at least about 95% sequence identity to SEQ ID NO:17. In some embodiments, the present invention is directed to an isolated, purified, synthetic, and/or recombinant polypeptide which consists essentially of or consists of a C-terminal sequence having about 95-100%, at least about 96%, at least about 97%, at least about 98%, or at least about 99% sequence identity to SEQ ID NO:16 and an N-terminal sequence having about 70-100%, at least about 80%, at least about 90%, or at least about 95% sequence identity to SEQ ID NO:17. The present disclosure
is directed to an isolated, purified, synthetic, and/or recombinant polypeptide which has about 70 to 100%, at least about 80%, at least about 90%, or at least about 95% sequence identity to SEQ ID NO:1 or SEQ ID NO:2.

The present disclosure is directed to an isolated, purified, synthetic, and/or recombinant polypeptide which comprises, consists essentially of, or consists of at least one of the following sequences: SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, and SEQ ID NO:15, wherein X is any amino acid. The present disclosure is directed to an isolated, purified, synthetic, and/or recombinant polypeptide which comprises, consists essentially of, or consists of all of the following sequences: SEQ ID NO:3, SEQ ID NO:4, SEQ ID NO:5, SEQ ID NO:6, SEQ ID NO:7, SEQ ID NO:8, SEQ ID NO:9, SEQ ID NO:10, SEQ ID NO:11, SEQ ID NO:12, SEQ ID NO:13, SEQ ID NO:14, and SEQ ID NO:15, wherein X is any amino acid.

The polypeptides described herein decrease hepatic hepcidin mRNA and/or serum hepcidin levels in human subjects when administered thereto.

The polypeptides described herein exhibit erythroferrone activity that is the same or substantially similar to that of SEQ ID NO:1 or SEQ ID NO:2. The polypeptides described herein exhibit erythroferrone activity that is about 60-100%, about 70-100%, about 80-100%, about 90-100%, or about 95-100% of that provided by SEQ ID NO:1 or SEQ ID NO:2.

The present disclosure is directed to an isolated, purified, synthetic, and/or recombinant polypeptide which comprises, consists essentially of, or consists of at least about 10, at least about 11, at least about 12, at least about 13, at least about 14, at least about 15, at least about 16, at least about 17, at least about 18, at least about 19, at least about 20, at least about 21, at least about 22, at least about 23, at least about 24, or at least about 25 consecutive amino acid residues of SEQ ID NO:1, SEQ ID NO:2, or SEQ ID NO:16.

The polypeptides described herein are made using synthetic or recombinant techniques.

The present disclosure is directed to an isolated, purified, synthetic, and/or recombinant nucleic acid molecule which comprises, consists essentially of, or consists of a sequence which encodes a polypeptide according to the present disclosure.

The present disclosure is directed to a recombinant cell which is capable of expressing a polypeptide according to the present invention and/or comprises a nucleic acid molecule which encodes a polypeptide according to the present disclosure.

The present invention is directed to an antibody which was raised against a polypeptide according to the disclosure. The antibody may be a monoclonal antibody. In some embodiments, the antibody is a humanized antibody, a chimeric antibody, or a human antibody.

In some embodiments, the present invention is directed to a composition which comprises, consists essentially of, or consists of
an antibody which was raised against a polypeptide according to the present disclosure. In some embodiments, the composition comprises
an antibody which was raised against a polypeptide according to the present disclosure in a concentration that is higher than that found in normal subjects.

The present invention is directed to the use of an antibody of the invention for treating a disease of iron metabolism in a subject.

In some embodiments, the subject is a human. In some embodiments, the subject is in need thereof. As used herein, a "subject in need" is one who has been diagnosed as having or suffers from a disease of iron metabolism. The disease of iron metabolism is an iron overload disease or a disease and/or disorder associated with abnormally low levels of hepcidin.

The polypeptide administered to the subject may be an -ERFE polypeptide. The polypeptide administered to the subject may be a +ERFE polypeptide. The one or more polypeptides may be administered in an effective amount, preferably a therapeutically effective amount. The method described herein may comprise, consist essentially of, or consist of regulating the amount of hepcidin in the subject by administering to the subject an ERFE polypeptide in an effective amount, preferably a therapeutically effective amount. In some embodiments, the treatment comprises measuring the amount of one or more ERFE polypeptides and/or hepcidin in the subject before, during, or after administration. In some embodiments, the amount of ERFE polypeptides is measured with an assay using one or more nucleic acid molecules which encode a polypeptide according to the present disclosure and/or one of more antibodies of the invention raised against a polypeptide according to the present disclosure. The methods described herein may comprise administering to the subject one or more +ERFE polypeptides where the measurement of hepcidin is above normal or one or more -ERFE polypeptides where the measurement of hepcidin is below normal.

The present disclosure is directed to an assay for detecting the presence of and/or measuring the amount of an ERFE polypeptide in a sample which comprises contacting the sample with an antibody raised against a polypeptide according to the present disclosure and then detecting the presence of and/or measuring the amount of bound antibodies.

In some embodiments, the present invention is directed to a kit comprising
an antibody raised against a polypeptide according to the present disclosure, or composition as disclosed herein packaged together with a reagent, a device, instructional material, or a combination thereof.

In some embodiments, the present invention is directed to a hybridoma cell line which is capable of expressing the antibody which specifically recognizes a polypeptide according to the present disclosure or raised against a polypeptide according to the present disclosure.

In some embodiments, the present invention is directed to a complex comprising at least one polypeptide according to the present disclosure bound to an antibody, wherein the at least one polypeptide and/or the antibody is made by recombinant techniques.

The present disclosure is directed to use of one or more polypeptides according to the present disclosure, a nucleic acid molecule which encodes a polypeptide according to the present disclosure, an antibody raised against a polypeptide according to the present disclosure, or a composition comprising one or more of the polypeptides, nucleic acid molecules, and antibodies for the manufacture of a medicament for treating a disease of iron metabolism.

The present invention is directed to an antibody raised against a polypeptide according to the present disclosure, or a composition comprising said antibodies for use in treating a disease of iron metabolism.

### DETAILED DESCRIPTION OF THE INVENTION

Both the foregoing general description and the following detailed description are exemplary and explanatory only and are intended to provide further explanation of the invention as claimed. The accompanying drawings are included to provide a further understanding of the invention and constitute part of this specification, illustrate several embodiments of the invention, and together with the description serve to explain the principles of the invention.

### DESCRIPTION OF THE DRAWINGS

This invention is further understood by reference to the drawings wherein:

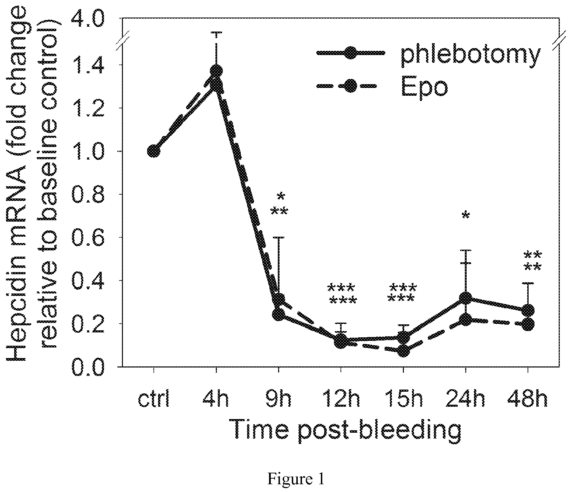

Figure 1: Hepatic *Hamp* mRNA is suppressed after erythropoietic stimulation (phlebotomy (solid line) or EPO injection (dashed line)) relative to untreated animals. Male 6-wks old mice were used, n=4/group. *p<0.05, **p<.01, ***p<.001, by Student t-test compared to untreated animals.

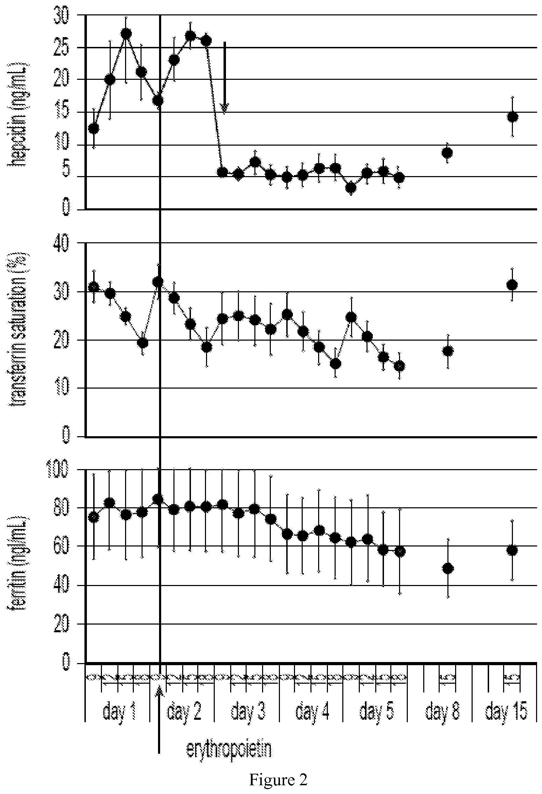

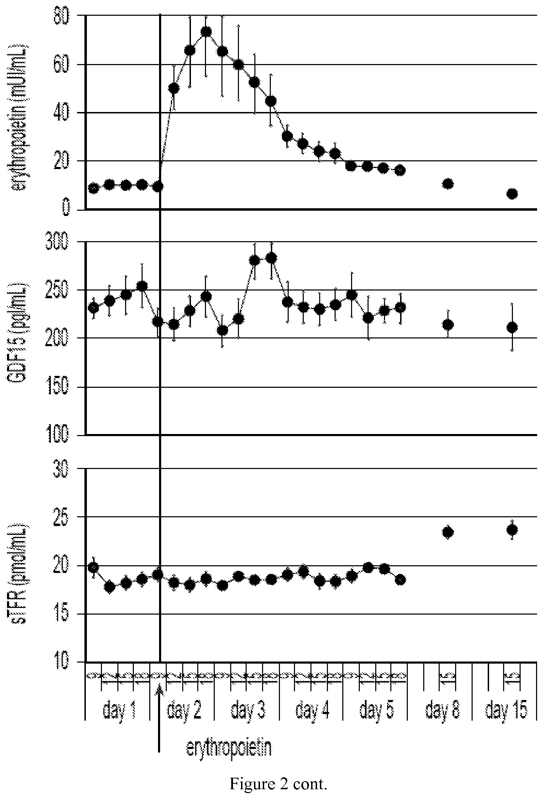

Figure 2: Responses of healthy human volunteers to EPO 5000 U given at 9 AM on day 2 (Ashby 2010).

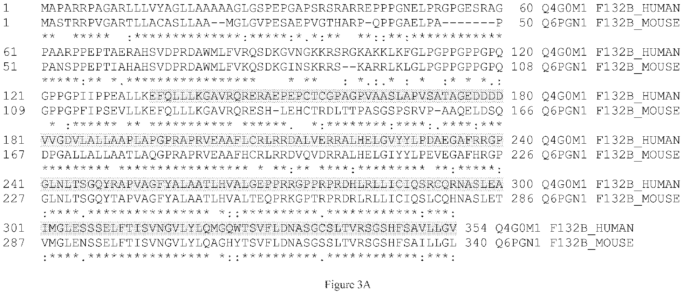

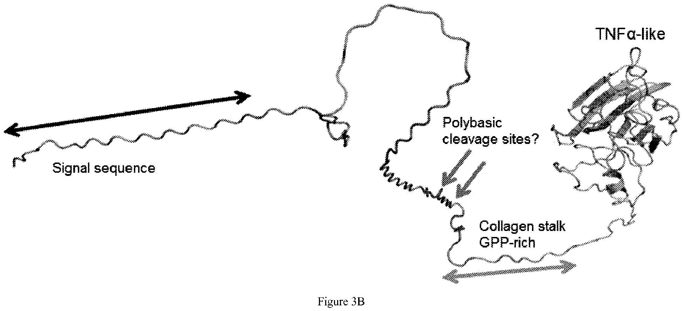

Figures 3A-3B: ERFE is a member of the TNF-α superfamily. Figure 3A: Alignment of mouse (SEQ ID NO:2) and a human ERFE protein (SEQ ID NO:1). The highlighted sequence is SEQ ID NO:16. Colored CLUSTAL alignments of the C-terminal segment of human ERFE to other human members of the TNF family and human variants of ERFE were performed. It was found that human variants of ERFE differ in signal sequence. Thus, ERFE polypeptides according to the present disclosure comprise 90-100%, preferably 95-100%, more preferably 98-100%, and most preferably 99-100% sequence identity to the N-terminal segment as set forth in SEQ ID NO:16. Figure 3B: A structural model of ERFE based on the similarities to other members of the TNFα superfamily.

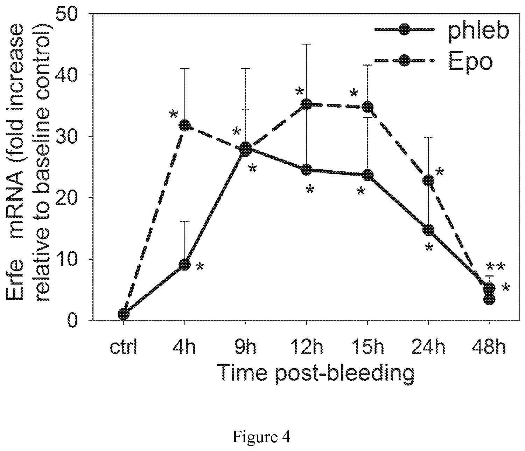

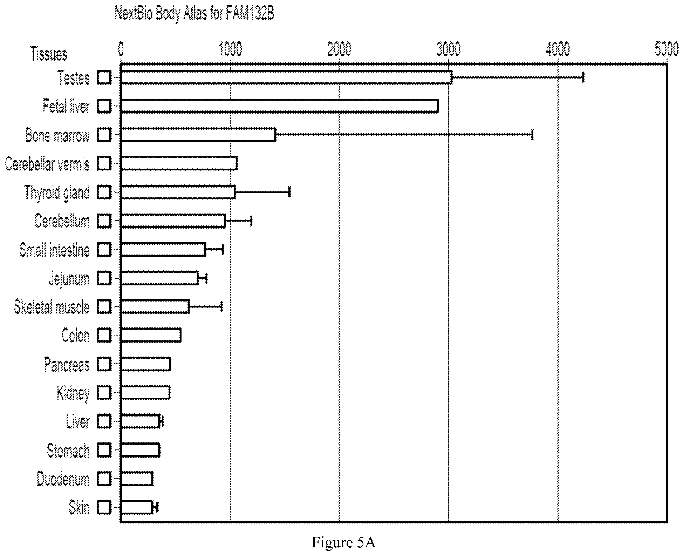

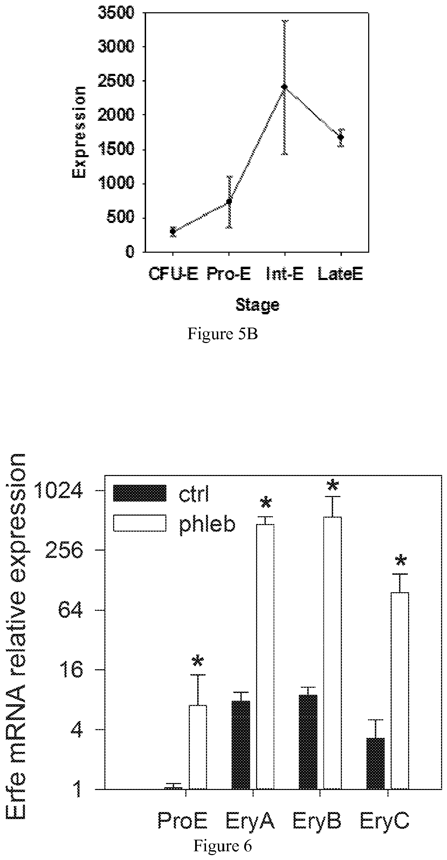

Figure 4: Fold increase in bone marrow *Erfe* mRNA after phlebotomy (solid line) or EPO injection (dashed line) compared to untreated mice. Six weeks old males were used, n=4/group. *p<.001, **p<.01 by t-test compared to untreated animals. Figure 5: Human *ERFE* is an erythroid transcript maximally expressed in intermediate erythroblasts. Figure 5A shows the relative human *ERFE* mRNA expression in various tissues in order of intensity. Fetal liver and bone marrow, organs that express human *ERFE* mRNA at a high level, contain erythroblasts. Figure 5B is based on Human Erythroblast Maturation Database (Merryweather-Clarke 2011). The relative expression of human *ERFE* mRNA in maturing human erythroblasts is shown, ranging from colony-forming units-erythroid (CFU-E), a very early stage of erythroid development, through proerythroblasts (Pro-E), intermediate erythroblasts (Int-E) to late erythroblasts (LateE).
Figure 6: *Erfe* mRNA expression in mouse marrow erythroblasts sorted into proerythroblast (ProE) and erythroblast EryA, B, C stages by the Socolovsky method (Liu 2006). White bars represent marrow from C57BL/6 mice 15 hours after phlebotomy of 0.5 ml; and black bars represent marrow from unmanipulated control mice. Six-week old male mice, n=3/group, *p<0.01 by t-test.

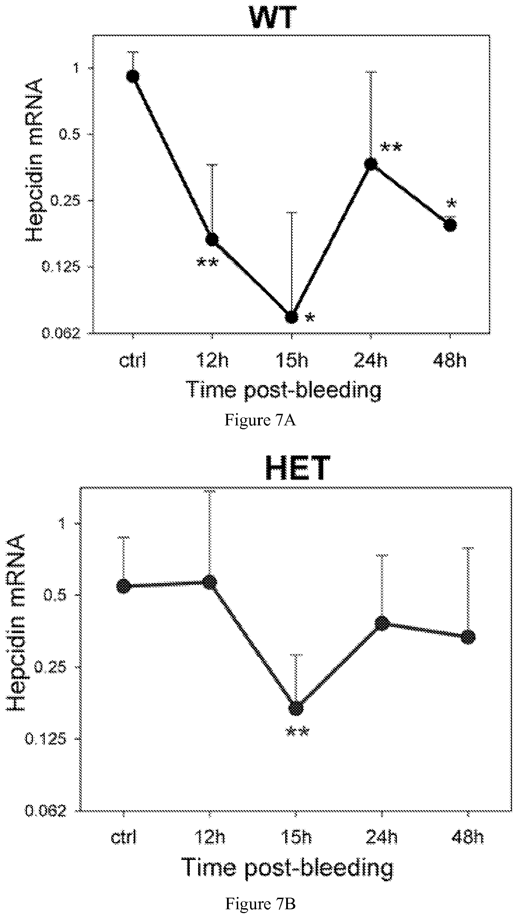

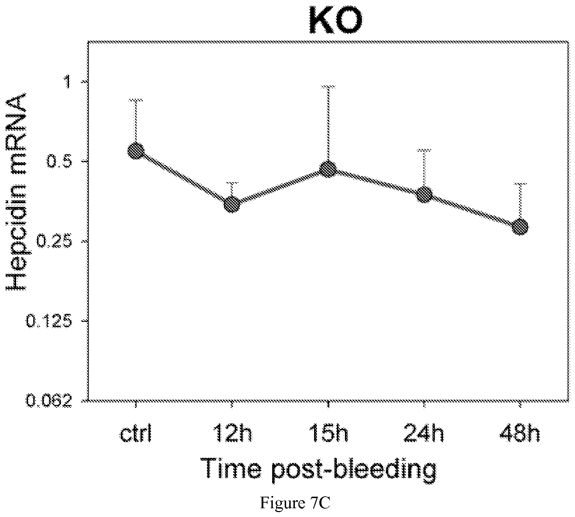

Figures 7A-7C: Change in *Hamp* mRNA in the liver of phlebotomized *Erfe*^{+/+}*, Erfe*^{+/-} and *Erfe*^{-/-} mice (labeled as WT (Wild-type, Figure 7A), HT (Heterozygote, Figure 7B), and KO (Knock Out, Figure 7C)). n=3 to 6 mice per group. *p<.001, **p<.01 by t-test compared to nonphlebotomized controls.

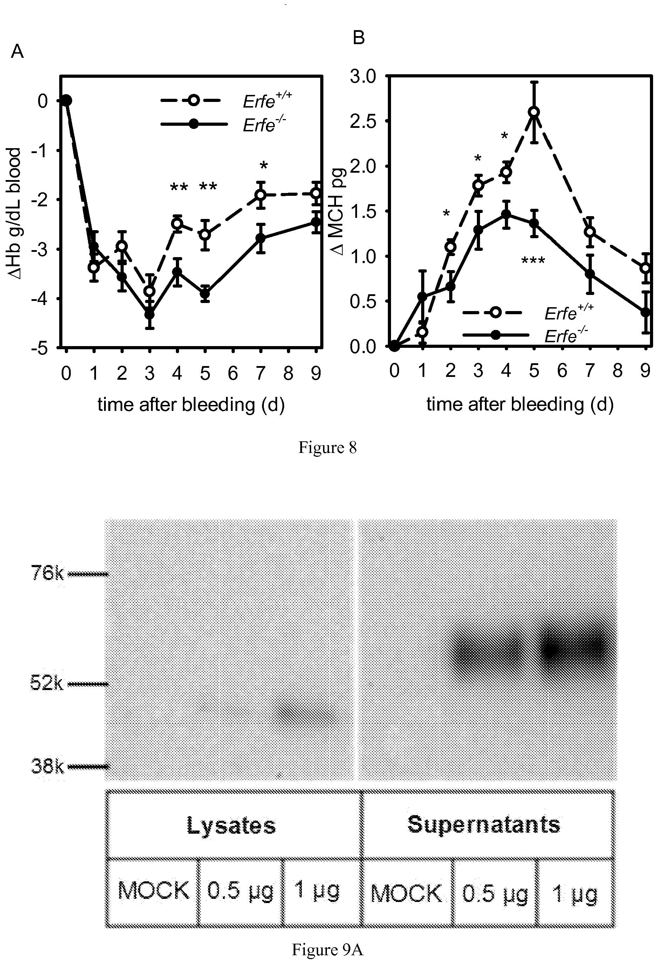

Figure 8: Erfe-deficient mice show delayed recovery from anemia. (A and B) Phlebotomized Erfe-deficient mice (solid line) compared to wild-type mice (dashed line) showed delayed recovery of hemoglobin and lower mean corpuscular hemoglobin (MCH). Hematological parameters (A, B) were compared for each measurement between WT (n=17) and KO (n=15) by student t-test. In the absence of gender differences, the genders were combined for each parameter. ***p<0.001, **p<0.01, *p<0.05.

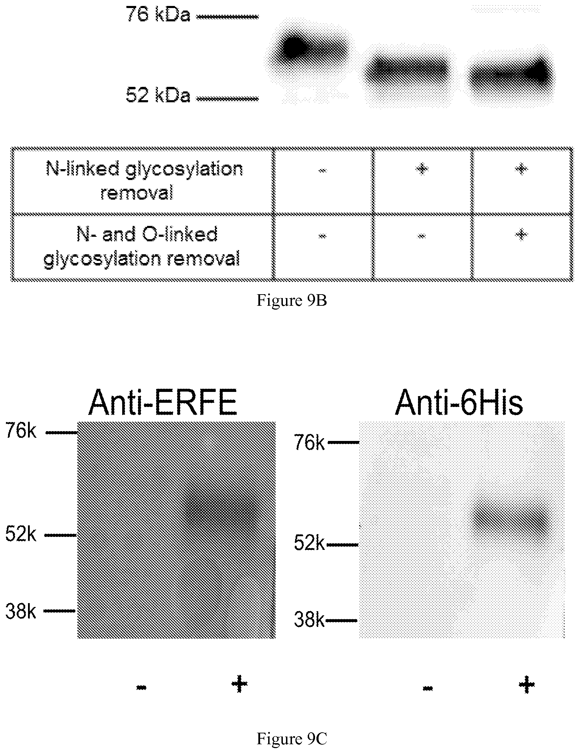

Figures 9A-9C: Figure 9A: HEK293T cells transfected with 0.5 or 1 µg of plasmid DNA encoding mouse *Erfe* cDNA fused to a C-terminal 6His tag. Western blot with anti-6His antibody detects Erfe predominantly in the supernatant indicating that Erfe is massively secreted. Figure 9B: Secreted Erfe is N- and O-glycosylated. Figure 9C: The rabbit anti-mouse anti- Erfe antibody 2 raised against an internal peptide sequence PSRVPAAQEL (SEQ ID NO:18) detects the recombinant protein in a Western blot, where +/- indicates transfection with *Erfe* plasmid or not.

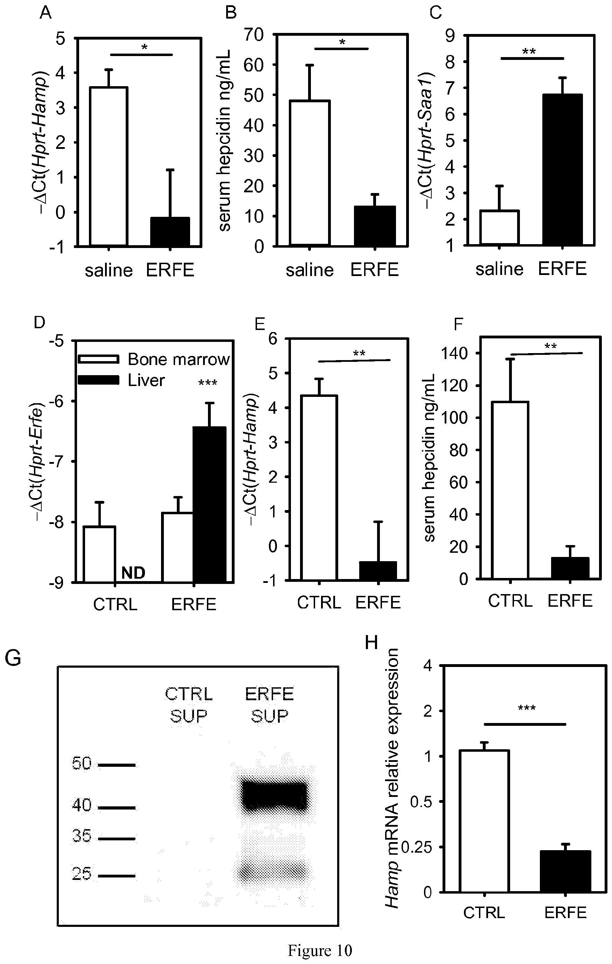

Figure 10: Erfe acts directly on the liver to suppress hepcidin. (A, B, C) Six C57BL/6 male mice were treated intraperitoneally with either mouse recombinant Erfe (2 µg/g) or saline and analyzed 15 hours later. Hepatic hepcidin mRNA (A) and serum hepcidin (B) levels were significantly suppressed by Erfe treatment despite the inflammatory response indicated by an increase in liver *Saa1* mRNA expression (C). (D, E, F) Eight 7 week-old C57BL/6 males were transduced with a lentivirus encoding GFP or mouse *Erfe* cDNA sequence. (D) *Erfe* mRNA expression was mildly increased in the liver of mice transduced with the *Erfe* lentivirus (only 2-fold higher than the baseline bone marrow level compared to a 32-fold upregulation in the bone marrow of mice with erythropoetic stimulation) but this increase was sufficient to reduce hepatic hepcidin mRNA (E) and serum hepcidin (F) levels. (G) Recombinant mouse Erfe is secreted by HEK293T cells infected with the *Erfe* lentivirus, as detected by Western blot with an anti-FLAG antibody. (H) Treatment of mouse primary hepatocytes with supernatants (50% v/v) from control cells or HEK293T cells overexpressing Erfe indicated that Erfe acts directly on the liver to suppress hepcidin mRNA expression. Means ± SEM of 3 independent experiments with 15 hours treatments in triplicate are shown. Hepcidin (*Hamp*)*,* serum amyloid 1 (*Saa1*) and *Erfe* mRNA (*Erfe*) levels were measured by qRT-PCR. Means ± SEM of -ΔCt (i.e., Ct Hprt - Ct *Hamp, Saa1* or *Erfe*) are shown. Serum hepcidin levels (B, F) were measured by enzyme-linked immunosorbent assay. Mean values were compared between treated mice and control mice or between Erfe-treated to control samples by Student t-test. ***p<0.001, **p<0.01, *p<0.05.

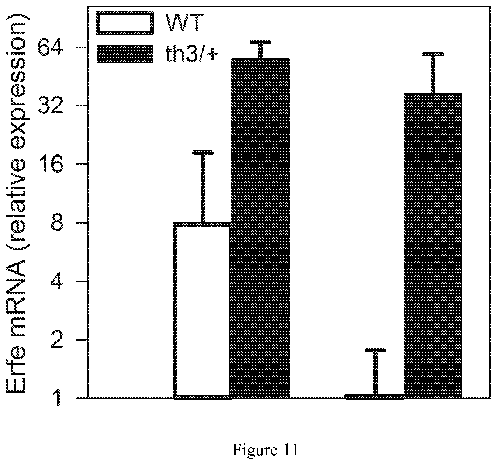

Figure 11: Increased *Erfe* mRNA in the bone marrow and spleen of β-thalassemia intermedia th3/+ mice (thai) (n=4) compared to wild-type mice (WT) (n=5). Male 6-month old mice were used. *p=0.016, **p<0.001. *Hprt* was used as the reference gene.

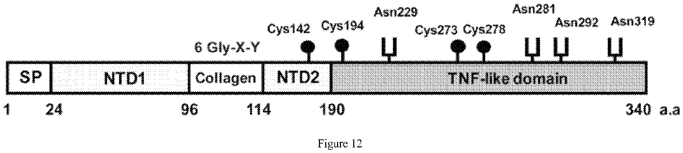

Figure 12: Predicted structural domains of Erfe. SP = signal peptide, NTD = N-terminal domains 1 and 2.

### DETAILED DESCRIPTION OF THE INVENTION

The present invention is based on the discovery of a protein, which is referred to herein as "Erythroferrone". Erythroferrone is the first identified "hormone" that mediates between red cell production and the absorption and distribution of iron in subjects. Erythroferrone is made in the marrow of a subject and its production is greatly increased when the production of red blood cells is stimulated, e.g., after bleeding or during recovery from anemia. Erythroferrone regulates the supply of iron to meet the needs of red cell production in the marrow. Specifically, erythroferrone is found to act on the liver to suppress the production of the principal iron-regulatory protein, hepcidin. Thus, overproduction of erythroferrone may cause iron overload in diseases such as β-thalassemia and antagonizing erythroferrone could thus be used for the treatment of β-thalassemia.

Erythroferrone was discovered in the search for a factor that suppresses hepcidin expression. Hepcidin, a 25 amino acid peptide hormone synthesized by the liver, is the central regulator of iron homeostasis. See Ganz 2011. Hepcidin acts by binding to the sole iron exporter ferroportin leading to its ubiquitination, internalization and degradation in lysosomes. When ferroportin disappears from the cell membranes, dietary absorption is inhibited and recycled iron is sequestered in macrophages, decreasing iron availability for erythropoiesis. In contrast, low hepcidin allows ferroportin to remain active on cells that export iron to plasma, making more iron available for hemoglobin synthesis. Iron, inflammation, or ER stress stimulates hepcidin production, whereas hypoxia, iron deficiency and increased erythropoietic activity repress it.

Hepcidin is suppressed after hemorrhage or erythropoietin (EPO) administration. Hepcidin is decreased in anemia caused by bleeding, hemolysis or iron deficiency, or in hereditary anemias with ineffective erythropoiesis. The suppressive effect of erythropoiesis on hepcidin is particularly prominent in diseases with ineffective erythropoiesis where erythrocyte precursors massively expand but mostly undergo apoptosis at the erythroblast stage rather than mature into erythrocytes. Thus, in C57BL/6 mice, the time-course of hepcidin mRNA in response to hemorrhage (500 µL) or EPO injection (200 U) was examined and a significant suppression of hepatic hepcidin mRNA between 9 and 15 hours was observed. At 12-15 hours after erythropoietic stimulation, the inhibition was maximal at about 90% (Figure 1). The timing of the response is similar to that in human volunteers where the decrease occurs between 9-24 hours (Figure 2).

This *in vivo* data on the time-course of response to hemorrhage in mice reveals that neither the transcription factor core element binding protein α(C/EBPα) mRNA nor the protein levels in the liver change, even though there is an observable potent suppression of hepcidin. Neither is the suppression of hepcidin after EPO administration due to increased iron utilization because the dramatic hepcidin decrease precedes any changes in transferrin saturation or other known iron-related parameters (Figure 2). Thus, in response to anemia or other hypoxic stimuli, it appears that high levels of EPO cause hepcidin suppression indirectly, by inducing the secretion of one or more erythroid factors from the bone marrow which, in turn, act on the liver to suppress hepcidin expression and increase iron delivery from dietary absorption and stores. The early suppression of hepcidin following EPO injection may make more iron available for rapid uptake by erythrocyte precursors, and accelerate erythropoiesis.

Thus, the gene expression profiles in the bone marrow in response to bleeding (0-48 hours), using commercially available gene chip-based expression profiling (Affymetrix Mouse Gene 1.0 ST Array) were studied. Expression profiling could also have been accomplished by alternative methods including next generation sequencing of cDNA referred to as "RNA Seq" in the art. Among the erythroid-specific transcripts whose expression was induced prior to the suppression of hepcidin mRNA and maintained at high levels before hepcidin level returned to normal baseline was a previously uncharacterized orphan transcript, listed in various public sequence databases as *Fam132b,* that encodes a secreted protein. *Fam132b* mRNA was found to be highly induced within 4 hours after bleeding, prior to hepcidin suppression. Erythroferrone is a protein encoded by *Fam132b* mRNA.

Except when referring to "ERFE polypeptides" (+ERFE and -ERFE polypeptides) and "ERFE polynucleotide"s, as used herein, "*ERFE*" (all capitals and italicized) refers to the human gene encoding erythroferrone, "*Erfe*" (first letter capitalized, and italicized) refers to the mouse gene encoding erythroferrone, "ERFE" (all capitals and non-italicized) refers to the human protein erythroferrone, and "Erfe" (first letter capitalized, and non-italicized) refers to the mouse protein erythroferrone.

*Erythroferrone is a member of the TNFα superfamily* - The amino acid sequence of erythroferrone homologs is well conserved through vertebrate evolution so that mouse and human proteins are about 71% identical (Figure 3A) and the C-terminal half is about 44% identical to the zebrafish homolog. Domain analysis indicated that Erfe is a member of the TNFα superfamily with only a moderate similarity to known cytokines. TNFα and the RANK ligand (RANKL) are the closest relatives. Search of the genomic and mRNA databases identified two variants of human ERFE that differ only in signal sequence. It remains to be determined whether both of these are expressed in the marrow and whether the variants give rise to proteins with different levels of expression or different biological activity. By reverse transcription of mRNA from human fetal liver-derived erythroblasts and cDNA sequencing, the C-terminal 220 amino acids (highlighted in Figure 3A) were confirmed.

Structural modeling of the entire protein using HHPredictB (Figure 3B) indicates that the N-terminal portion of the protein (left) consists of a signal sequence followed by an open region with a collagen-like segment and the C-terminal portion is homologous to TNFα/RANKL (right). The similarities to TNFα are remarkable as TNFα suppresses hepcidin mRNA in primary hepatocyte cultures. The similarity to TNFα predicts a tendency to form multimers.

*Erythroferrone expression in the marrow responds to EPO* - Using qPCR, the induction of *Erfe* mRNA expression following phlebotomy in the bone marrow (Figure 4) as well as in the spleen (data not shown) was confirmed, thereby supporting its involvement in erythropoiesis. EPO injection in mice leads to comparable hepcidin suppression as in phlebotomized mice, both in amplitude and time course (Figure 1). Likewise, the response of *Erfe* mRNA to EPO in the bone marrow and the spleen is similar to that caused by phlebotomy but *Erfe* stimulation occurs earlier in EPO-injected mice (Figure 4). This is consistent with Erfe acting downstream of EPO in the phlebotomy model.

*Erythroferrone is likely an erythrokine produced by erythroblasts* - Search of gene expression databases revealed that human *ERFE* is highly expressed in the human bone marrow and the fetal liver (e.g., Figure 5A), and in "*in vitro"* differentiated human CD34+ cells reaches maximum expression in intermediate erythroblasts, with an approximately 8-fold increase over CFU-E (Figure 5B). This suggests that erythroblasts are the main source of erythroferrone. This was directly verified with *ex vivo* mouse bone marrow obtained 15 hours after 0.5 ml phlebotomy, which showed a massive increase of *Erfe* expression in erythroblasts compared to control mice, predominantly in the (early and intermediate erythroblast) EryA and B stages (Figure 6, qRT-PCR, *Hprt* reference). Because of the relative abundance of the EryB stage, these cells likely constitute the main source of Erfe in the marrow.

*Hepcidin response to hemorrhage in Erfeknockout and haploinsufficient mice - Erfe*^{+/-} mice on a mixed Sv129/C57BL/6 background (Fam132btm1Lex, Lexicon Pharmaceuticals) as well as those backcrossed onto the C57BL/6 background using marker-assisted accelerated backcrossing may be used to further analyze Erfe polypeptides according to the present disclosure. In some experiments, the mixed background *Erfe^{-l-}* and *Erfe*^{+/-} were compared to their wild-type (WT) littermates. *Erfe* knockouts (KO) are viable, appear phenotypically normal, have a completely normal and very extensive standardized workup done for the Lexicon mouse database, and are fertile. Complete blood counts performed using an automated hematology analyzer (Hemavet 850; Drew Scientific, Oxford, CT) at 6 weeks of age (but not 3 weeks, 12 weeks or 6 months) revealed decreased hemoglobin levels at baseline in Erfe-deficient animals compared to wild-type and heterozygotes. The age 6 weeks is the time of rapid erythroid expansion and growth with high demand for iron, similar to stress erythropoiesis after bleeding. To test whether Erfe is a critical mediator of hepcidin repression by bleeding, *Erfe*^{-/-}*, Erfe*^{+/-} and *Erfe*^{+/+} littermates were phebotomized (500 µL) and analyzed 12, 15, 24 and 48 hours after phlebotomy (Figures 7A-7C). *Erfe*^{-/-} mice failed to decrease hepatic hepcidin mRNA expression indicating that *Erfe* is essential for rapid hepcidin suppression after hemorrhage (Figures 7A-7C). The haploinsufficent mice showed only a partial response at 12-15 hours suggesting a dose-dependent response of hepcidin to Erfe.

*Erfe-deficient mice show delayed recovery from bleeding-induced anemia-*Twelve week-old *Erfe*^{+/+} and *Erfe* ^{-/-} were phlebotomized and their recovery was monitored during 9 days. Erfe -deficient mice exhibited lower hemoglobin and mean corpuscular hemoglobin compared to wild-type controls (Figure 8) and the recovery of Erfe-deficient mice from hemorrhage-induced anemia was delayed by several days.

*Recombinant protein production and analysis* - Multiple expression plasmids encoding the cDNA of mouse and human ERFE with a C-terminal Myc/Flag, V5 epitope/His, Myc/His and Flag/His were constructed. Western blotting of transfected HEK293T confirmed that the protein is secreted (Figure 9A). The material was purified by tag affinity chromatography, analyzed on SDS-PAGE, and the major bands identified corresponding to those detected by Western blotting. The identity of the bands as Fam132b/Erfe was confirmed by proteomic analysis of trypsinized fragments by HPLC-MS. Interestingly, the secreted form of Erfe has a slightly higher molecular weight than the cellular form, and both proteins were twice the predicted size, indicating possible dimerization. Deglycosylation of the purified secreted product indicated that Erfe is N- and O-glycosylated (Figure 9B). Recombinant forms of Erfe and Erfe polypeptides may be used to analyze their activities *in vitro* and *in vivo.* Antibodies raised against Erfe were generated and can be used for endogenous protein detection (Figure 9C) and to determine serum concentrations of one or more ERFE polypeptides in subjects, e.g., mouse models and patients with iron disorders. In preliminary experiments, one variant of the HEK293 cell line was found to express Erfe at high levels but failed to secrete it, thereby indicating that the secretion event may be cell-type specific or dependent on specialized features of the cell.

To test whether secreted erythroferrone protein is a suppressor of hepcidin, C57BL/6 male mice were injected with murine recombinant Erfe (2 µg/g). Hepcidin mRNA and serum protein levels were found to be significantly reduced 15 hours after Erfe treatment compared to saline-injected mice (Figures 10A, 10B) despite a substantial inflammatory response triggered by the recombinant protein as shown by increased expression of *Saa1* mRNA (Figure 10C). Inflammation would increase hepcidin expression.

To avoid the acute inflammatory effect of the recombinant Erfe preparation, C57BL/6 male mice were transduced with a lentiviral-based vector encoding Erfe. Three weeks later, a modest increase of *Erfe* mRNA was observed in the liver (but not the bone marrow) of mice treated with *Erfe*-lentivirus compared to mice treated with the control lentivirus (Figure 10D). Nevertheless, hepcidin mRNA and serum levels were significantly reduced 30-fold and 10-fold respectively (Figures 10E, 10F) suggesting that even a small amount of Erfe in the liver was sufficient to exert its inhibitory effect on hepcidin transcription. Therefore, whether Erfe could act directly on the liver to regulate hepcidin was tested. Mouse primary hepatocytes were treated for 15 hours with 50% (v/v) supernatants from control HEK293T or HEK293T overexpressing Erfe (Figure 10G), resulting in a 4-fold decrease in hepcidin mRNA levels in Erfe-treated cells (Figure 10H). Taken altogether, these results show that erythroferrone is a potent suppressor of hepcidin that can act directly on the liver to repress hepcidin mRNA expression.

*Preparation of antibodies* - To facilitate the detection and characterization of erythroferrone, a series of antibodies directed against peptide antigens in both the murine and human forms of erythroferrone and ERFE polypeptides may be generated and used. In addition, antibodies against the internal and N-terminal epitopes of erythroferrone and/or ERFE polypeptides may be used in various assays and treatments according to the present invention.

*Massive increase of Erfe in thalassemic mice* - Erythroferrone overproduction could contribute to the iron overload of nontransfused β-thalassemia. Using a Hbb^{th3/+} mouse model of β-thalassemia (Gardenghi 2007), it was shown that *Erfe* mRNA is greatly increased in the marrow and spleen of Hbb^{th3/+} mice compared to wild-type controls (Figure 11). The net expression of *Erfe* is even greater in Hbb^{th3/+} mice if one takes into account that they have expanded marrow and 3x larger spleens than wild-type mice.

### Polypeptides and Compositions

The present disclosure is directed to erythroferrone and analogs and fragments thereof. As used herein, erythroferrone (including ERFE and Erfe) and its analogs and fragments are collectively referred to herein as "ERFE polypeptides". ERFE polypeptides may or may not exhibit erythroferrone activity. As used herein, "erythroferrone activity" refers to the ability of the substance to decrease hepatic hepcidin mRNA or serum hepcidin levels as compared to a control.

As used herein, the terms "protein", "polypeptide" and "peptide" are used interchangeably to refer to two or more amino acids linked together. Groups or strings of amino acid abbreviations are used to represent peptides. Except when specifically indicated, peptides are indicated with the N-terminus on the left and the sequence is written from the N-terminus to the C-terminus.

The ERFE polypeptides of the present disclosure may be made using methods known in the art including chemical synthesis, biosynthesis or *in vitro* synthesis using recombinant DNA methods, and solid phase synthesis. See e.g., Kelly & Winkler (1990) Genetic Engineering Principles and Methods, vol. 12, J. K. Setlow ed., Plenum Press, NY, pp. 1-19; Merrifield (1964) J Amer Chem Soc 85:2149; Houghten (1985) PNAS USA 82:5131-5135; and Stewart & Young (1984) Solid Phase Peptide Synthesis, 2ed. Pierce, Rockford, IL. The ERFE polypeptides of the present disclosure may be purified using protein purification techniques known in the art such as reverse phase high-performance liquid chromatography (HPLC), ion-exchange or immunoaffinity chromatography, filtration or size exclusion, or electrophoresis. See Olsnes and Pihl (1973) Biochem. 12(16):3121-3126; and Scopes (1982) Protein Purification, Springer-Verlag, NY.

Alternatively, the ERFE polypeptides of the present disclosure may be made by recombinant DNA techniques known in the art. Thus, polynucleotides that encode the ERFE polypeptides of the present disclosure are contemplated herein and are herein referred to as "ERFE polynucleotides".

The ERFE polynucleotides may be isolated. As used herein, "isolated" polynucleotides refers to polynucleotides that are in an environment different from that in which the polynucleotide naturally occurs. For example, an isolated polynucleotide is a one which does not have the bases normally flanking the 5'- and/or 3' ends of the polynucleotide as it is found in nature.

The ERFE polypeptides of the present disclosure are substantially purified. As used herein, a "substantially purified" compound refers to a compound that is removed from its natural environment and/or is at least about 60% free, preferably about 75% free, and more preferably about 90% free, and most preferably about 95-100% free from other macromolecular components or compounds with which the compound is associated with in nature or from its synthesis.

As used herein, an "isolated" compound refers to a compound which is isolated from its native environment. For example, an isolated polypeptide is a one which does not have its native amino acids, which correspond to the full length polypeptide, flanking the N-terminus, C-terminus, or both. For example, an isolated fragment of erythroferrone or an ERFE polypeptide refers to an isolated polypeptide that comprises some, but not all, of the amino acid residues erythroferrone or the ERFE polypeptide, but may have non-native amino acids at its N-terminus, C-terminus, or both, i.e., different amino acids that do not have identity to the amino acids at the corresponding position of the erythroferrone or the ERFE polypeptide.

The ERFE polypeptides of the present disclosure are made using synthetic and/or recombinant techniques.

One or more ERFE polypeptides, as described herin, may be provided in the form of a composition which comprises a carrier suitable for its intended purpose. The compositions may also include one or more additional ingredients suitable for its intended purpose. For example, for assays, the compositions may comprise liposomes, niclosamide, SL220 solubilization agent (NOF, Japan), Cremophor EL (Sigma), ethanol, and DMSO. For treatment of an iron disorder, the compositions may comprise different absorption enhancers and protease inhibitors, solid microparticles or nanoparticles for peptide encapsulation (such as chitosan and hydrogels), macromolecular conjugation, lipidization and other chemical modification. The compositions of the present disclosure comprise one or more ERFE polypeptides at a concentration that is higher than that as found in normal subjects.

One or more ERFE polypeptides according to the present disclosure or compositions thereof may be used to treat diseases of iron metabolism. As used herein, a "disease of iron metabolism" includes diseases where aberrant iron metabolism directly causes the disease, or where iron blood levels are dysregulated causing disease, or where iron dysregulation is a consequence of another disease, or where diseases can be treated by modulating iron levels, and the like. More specifically, a disease of iron metabolism according to this disclosure includes iron overload diseases, iron deficiency disorders, disorders of iron biodistribution, other disorders of iron metabolism and other disorders potentially related to iron metabolism, etc. Diseases of iron metabolism include hemochromatosis, HFE mutation hemochromatosis, ferroportin mutation hemochromatosis, transferrin receptor 2 mutation hemochromatosis, hemojuvelin mutation hemochromatosis, hepcidin mutation hemochromatosis, juvenile hemochromatosis, neonatal hemochromatosis, hepcidin deficiency, transfusional iron overload, thalassemia, thalassemia intermedia, alpha thalassemia, sideroblastic anemia, porphyria, porphyria cutanea tarda, African iron overload, hyperferritinemia, ceruloplasmin deficiency, atransferrinemia, congenital dyserythropoietic anemia, anemia of chronic disease, anemia of inflammation, anemia of infection, hypochromic microcytic anemia, iron-deficiency anemia, iron-refractory iron deficiency anemia, anemia of chronic kidney disease, erythropoietin resistance, iron deficiency of obesity, other anemias, benign or malignant tumors that overproduce hepcidin or induce its overproduction, conditions with hepcidin excess, Friedreich ataxia, gracile syndrome, Hallervorden-Spatz disease, Wilson's disease, pulmonary hemosiderosis, hepatocellular carcinoma, cancer, hepatitis, cirrhosis of liver, pica, chronic renal failure, insulin resistance, diabetes, atherosclerosis, neurodegenerative disorders, multiple sclerosis, Parkinson's disease, Huntington's disease, and Alzheimer's disease. In some embodiments, the iron overload disease is myelodysplastic syndrome. In some cases the diseases and disorders included in the definition of "disease of iron metabolism" are not typically identified as being iron related. For example, hepcidin is highly expressed in the murine pancreas suggesting that diabetes (Type I or Type II), insulin resistance, glucose intolerance and other disorders may be ameliorated by treating underlying iron metabolism disorders. See Ilyin, G. et al. (2003) FEBS Lett. 542 22-26.

As such, these diseases are encompassed under the broad definition. Those skilled in the art are readily able to determine whether a given disease is a "disease or iron metabolism" according to the present invention using methods known in the art, including the assays of WO 2004092405
and assays which monitor hepcidin, hemojuvelin, or iron levels and expression, which are known in the art such as those described in U.S. Patent No. 7,5344,764. In some embodiments of the present invention, the diseases of iron metabolism are iron overload diseases, which include hereditary hemochromatosis, iron-loading anemias, alcoholic liver diseases and chronic hepatitis C.

As used herein, diseases and disorders related to hepcidin excess include anemia of chronic disease (also called anemia of inflammation), anemias associated with acute or chronic infections, anemia of chronic kidney disease, anemias due to tumors that secrete hepcidin, and iron-refractory iron-deficiency anemia (IRIDA).

As used herein, diseases and disorders related to hepcidin deficit include adult and juvenile forms of hereditary hemochromatosis, α-thalassemia, β-thalassemia and congenital dyserythropoietic anemias, and chronic liver diseases including alcoholic liver disease and chronic hepatitis B and C.

As used herein, a "+ERFE polypeptide" refers to an ERFE polypeptide that exhibits erythroferrone activity that is the same or similar to that of mouse and/or human erythroferrone. Erythroferrone is a +ERFE polypeptide. As used herein, a "-ERFE polypeptide" refers to an ERFE polypeptide that does not exhibit erythroferrone activity and in some embodiments interferes with the activity of +ERFE polypeptides. Erythroferrone is not a -ERFE polypeptide. Thus, the term "ERFE polypeptides" is used to refer to both +ERFE polypeptides and -ERFE polypeptides. In other words, an ERFE polypeptide may be a +ERFE polypeptide or a -ERFE polypeptide.

### +ERFE Polypeptides and Treatments

The present disclosure is directed to methods of using one or more +ERFE polypeptides alone or in combination with one or more agonists of erythroferrone to treat diseases and disorders related to hepcidin excess and/or iron deficits, e.g., anemia of inflammation and iron-refractory iron deficiency anemia (IRIDA). The +ERFE polypeptides of the present disclosure may mimic the action of erythroferrone, i.e., exhibit the same or substantially similar activity to that of erythroferrone. The present disclosure is directed to methods of using one or more +ERFE polypeptides to inhibit, suppress or reduce hepcidin expression or prevent further hepcidin expression.

### -ERFE Polypeptides and Treatments

The present disclosure is directed to methods of using one or more -ERFE polypeptides alone or in combination with one or more antagonists of erythroferrone to treat diseases and disorders related to hepcidin deficits and/or iron overload. The present disclosure is directed to using one or more -ERFE polypeptides to increase hepcidin expression and/or amounts. The - ERFE polupeptides may include those which competitively compete with erythroferrone for the receptor for erythroferrone, and increase hepcidin expression and/or hepcidin amounts. Thus, in some instances, the present disclosure is directed to methods of using one or more -ERFE polypeptides alone or in combination with one or more antagonists of erythroferrone to treat diseases and disorders caused by hepcidin deficits, e.g., β-thalassemia or congenital dyserythropoietic anemia, and iron toxicity.

Thus, one or more ERFE polypeptides of the present disclosure may be administered to a subject, preferably a mammal such as a human.

The ERFE polypeptides may be administered in a form of a pharmaceutical composition. In some instances, the ERFE polypeptides are administered in a therapeutically effective amount. As used herein, a "therapeutically effective amount" is an amount which ameliorates the symptoms and/or pathology of a given disease of iron metabolism as compared to a control such as a placebo.

A therapeutically effective amount may be readily determined by standard methods known in the art. The dosages to be administered can be determined by one of ordinary skill in the art depending on the clinical severity of the disease, the age and weight of the subject, or the exposure of the subject to iron. Preferred effective amounts of the compounds of the invention ranges from about 0.01 to about 10 mg/kg body weight, preferably about 0.1 to about 3 mg/kg body weight, and more preferably about 0.5 to about 2 mg/kg body weight for parenteral formulations. Preferred effective amounts for oral administration would be up to about 10-fold higher. Moreover, treatment of a subject with an ERFE polypeptide or composition of the present disclosure can include a single treatment or, preferably, can include a series of treatments. It will be appreciated that the actual dosages will vary according to the particular ERFE polypeptide or composition, the particular formulation, the mode of administration, and the particular site, host, and disease being treated. It will also be appreciated that the effective dosage used for treatment may increase or decrease over the course of a particular treatment. Optimal dosages for a given set of conditions may be ascertained by those skilled in the art using conventional dosage-determination tests in view of the experimental data for a given ERFE polypeptide or composition. Changes in dosage may result and become apparent by standard diagnostic assays known in the art. In some conditions chronic administration may be required.

The pharmaceutical compositions of the invention may be prepared in a unit-dosage form appropriate for the desired mode of administration. The compositions of the present invention may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the condition to be treated, and the chosen ERFE polypeptide and composition.

Pharmaceutical compositions of the present invention may comprise a therapeutically effective amount of at least one ERFE polypeptide as disclosed herein, and an inert, pharmaceutically acceptable carrier or diluent. As used herein the language "pharmaceutically acceptable carrier" is intended to include any and all solvents, dispersion media, coatings, antibacterial and antifungal agents, isotonic and absorption delaying agents, and the like, compatible with pharmaceutical administration and known in the art. Except insofar as any conventional media or agent is incompatible with the active compound, use thereof in the compositions is contemplated.

Supplementary active compounds can also be incorporated into the compositions. Supplementary active compounds include niclosamide, liposomes, SL220 solubilization agent (NOF, Japan), Cremophor EL (Sigma), ethanol, and DMSO.

Toxicity and therapeutic efficacy of the ERFE polypeptides and compositions of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. ERFE polypeptides which exhibit large therapeutic indices are preferred. While ERFE polypeptides that exhibit toxic side effects may be used, care should be taken to design a delivery system that targets such ERFE polypeptides to the site of affected tissue in order to minimize potential damage to uninfected cells and, thereby, reduce side effects.

The data obtained from the cell culture assays and animal studies can be used in formulating a range of dosage for use in humans. The dosage of ERFE polypeptides of the present disclosure lies preferably within a range of circulating concentrations that include the ED₅₀ with little or no toxicity. The dosage may vary within this range depending upon the dosage form employed and the route of administration utilized. For any ERFE polypeptide used in the method of the disclosure, the therapeutically effective dose can be estimated initially from cell culture assays. A dose may be formulated in animal models to achieve a circulating plasma concentration range that includes the IC₅₀ (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

The present invention also provides kits comprising one or more compositions of the present invention packaged together with reagents, devices, instructional material, or a combination thereof. For example, the kits may include reagents used for conducting assays, drugs and compositions for diagnosing, treating, or monitoring disorders of iron metabolism, devices for obtaining samples to be assayed, devices for mixing reagents and conducting assays, and the like.

The following examples are intended to illustrate but not to limit the invention.

### EXPERIMENTS

### 1. The role of Erythroferrone in the erythron-dependent regulation of iron metabolism

Erythroferrone Production - Preliminary data indicates that *Erfe* mRNA was greatly increased in the bone marrow and the spleen after hemorrhage and EPO injection. The bone marrow and splenic cell types expressing *Erfe* transcript under normal conditions and during stress erythropoiesis were identified as follows: Briefly, total spleen and bone marrow cells are isolated from control mice and phlebotomized mice (15 hours), and sorted by flow cytometry based on the expression levels of Ter119, CD71 and forward scatter. See e.g., Socolovsky 2007; Chen 2009. *Erfe* mRNA expression was quantified by qPCR in each cell population relative to two housekeeping genes. Erfe expression massively increased in all erythroblast stages after phlebotomy compared to control mice (Figure 6) (ProE, EryA, B and C). Because of the relative abundance of the EryB stage, these cells likely constitute the main source of erythroferrone in the marrow. To ascertain whether Erfe is indeed an erythroid-specific product, qPCR analysis of representative mouse tissues at baseline and after phlebotomy can be performed.

*Hematopoietic cells* - To examine the contribution of hematopoietic cells to bioactive erythroferrone production, reciprocal bone marrow transplantation between *Erfe^{-l-}* and *Erfe*^{+/+} mice, after myeloablative irradiation, may be conducted using autotransplants as controls. Such experiments may be performed in accordance with recent veterinary guidelines. See Duran-Struuck 2009. After allowing 3 weeks for erythropoietic recovery and cessation of inflammatory reaction to radiation and transplant, suppression of hepcidin 15 hours after 500 µl phlebotomy can be used as a phenotypic readout. Engraftment and donor cell dominance can be confirmed by qPCR of the marrow DNA for the intact versus disrupted *Erfe* alleles, and the production of Erfe can be assessed by qRT-PCR (or ELISA, and the like). It is expected that the phenotype will be transferred with the marrow. In the unlikely alternative outcome, other cell types may be assayed as the source for erythroferrone.

*Regulation by EPO* - EPO may have dual effects on erythroferrone expression: 1) it increases the number of cells (intermediate erythroblasts) that express erythroferrone by promoting the survival of the precursors through the proerythroblast-basophilic erythroblast stages, or 2) it may directly or indirectly regulate erythroferrone mRNA concentrations. Because *Erfe* is near maximally, 30-fold, increased already 4 hours after EPO injection in mice (Figure 4), by which time the number of splenic or marrow erythroid progenitors increases only slightly, the second mechanism predominates, and the response to EPO is probably erythroblast-autonomous. Nevertheless, the regulation by EPO may be determined by the following: Briefly, freshly harvested mouse bone marrow cells are incubated for 4 hours supplementing the medium with concentrations of EPO from 0-2.5 u/ml and then will sort the erythroid cells using methods known in the art. Each erythroid maturation stage will be analyzed by qRT-PCR for *Erfe* expression as a function of EPO dose.

Because erythroferrone is not required for basal erythropoiesis, it is expected that erythroferrone will be regulated by the EPO receptor (EPOR) stress pathway. Stat5 is the main transcription factor of the EPO-EPOR/Jak2 pathway for stress erythropoiesis-activated genes (Teglund 1998; Menon 2006) and the analysis of 3 kilobases of the mouse and human erythroferrone promoter using Genomatix software showed multiple potential Stat5 (or other Stat) binding elements and GATA-binding motifs. Therefore, one may check if *Erfe* transcription is regulated by the Stat pathway according to the following: Erythroid precursors from mouse fetal liver and mouse marrow are cultured (England 2011), treated with graded concentrations of EPO for 4 hours with or without Stat5 inhibitors (pimozide or N'-((4-Oxo-4H-chromen-3-yl)methylene)nicotinohydrazide) and the effect on *Erfe* mRNA levels is assessed by qPCR. If erythroblasts tolerate the brief exposure to Stat5 inhibitors poorly, one may use the UT7 cell line that undergoes erythroid differentiation under EPO treatment or the continuous CD36E cell line, an erythroid progenitor cell line possessing the ability to produce hemoglobin (Wong 2010) or J2E cells line (Greene 2002). Using these cell lines and an *Erfe* promoter luciferase construct site-directed mutagenesis of putative Stat5 binding sites is performed to test the mechanism by which EPO regulates the *Erfe* promoter. Finally, if these approaches do not succeed in determining whether the STAT5 pathway regulates *Erfe* expression, one can obtain mice EPOR-HM mice deficient in the graded STAT5 response and determine whether these mice regulate *Erfe* in response to EPO. See Porpiglia 2012. Although less likely, one or more alternative pathways of EPO receptor signaling could be more important than STAT5 in regulating *Erfe.* In general, these pathways are less well understood but include the PI3-kinase and MAP-kinase pathways, and are predominantly involved in the regulation of erythroblast survival and proliferation. See Richmond 2005; Bouscary 2003.

*EPO dose dependence* - Therapeutic doses of EPO lower hepcidin concentration even under conditions of normal or increased endogenous EPO production, and even in the face of inflammation and iron overload. See Huang 2009. This important effect of EPO is not understood, and is important because it may contribute to its therapeutic effectiveness in anemia of inflammation and anemia of cancer where hepcidin is elevated and iron is sequestered in macrophages. The dose dependence of the response of liver hepcidin mRNA and serum hepcidin to graded doses of EPO (single injection, 0.2-200 U) in *Erfe* KO and heterozygote animals compared to WT controls can be determined. As shown in the preliminary data in WT mice, EPO injection had an identical effect as a single phlebotomy on the time-course of hepcidin or *Erfe* mRNA expression, so it is expected that EPO-injected *Erfe*^{*-*/-} mice will fail to suppress hepcidin similarly to the phlebotomized *Erfe*^{*-*/}*⁻.* It is possible that an alternative pathway suppressing hepcidin will become manifest in *Erfe^{-l-}* KO mice at higher concentrations of EPO. Although a direct effect of EPO on hepcidin expression was reported (Pinto 2008), such suppression was not observed in experiments by the instant inventors. If high dose EPO suppresses hepcidin in *Erfe*^{*-*/*-*} mice one may reexamine whether high concentrations of EPO can suppress hepcidin mRNA in the liver indirectly, perhaps by acting on nonhepatocyte cell populations that may also be admixed in some primary hepatocyte cultures.

*Anemia from hemolysis* - The phenylhydrazine-induced hemolysis model is traditionally used for studies of stress erythropoiesis in mice, and the anemia is associated with hepcidin suppression (Nicolas 2002). The model differs from hemorrhagic anemia in that recovery depends on heme and iron recycling. The effect of *Erfe* ablation on recovery from acute hemolytic anemia after a single dose of phenylhydrazine 100 mg/kg (Lim 1998) can be analyzed. Mice will be analyzed on days 2, 4 and 6 for complete blood count, erythroid maturation by flow cytometry, hepcidin expression, and iron parameters. It is expected that hepcidin will not be suppressed in *Erfe*^{*-*/*-*} mice and they will have a delayed erythroid recovery.

*Compensatory dietary iron absorption and iron release from stores* - In humans, dietary iron absorption accounts for less than 10% of the total daily iron needs and most iron is derived from macrophages recycling senescent erythrocytes. In mice fed standard chow, however, dietary absorption provides nearly 50% of the total daily iron requirement. See Ramos 2011. Thus, in order to differentiate if *Erfe* ablation has distinct effects on utilization of dietary versus recycled iron during recovery from hemorrhage, mouse iron stores and dietary iron content may be manipulated. To assess the effect on Erfe deficiency on dietary iron absorption, one first depletes iron stores by feeding the animals 4 ppm diet for two weeks. Once the iron stores are low as confirmed by baseline analysis of liver and spleen iron in a subgroup of mice, another group of animals are phlebotomized 500 µl and placed on an iron-replete (300 ppm) diet. To assess the effect of Erfe on iron recycling and mobilization from stores, mice are pretreated with iron dextran to provide them with sufficient iron stores to compensate for 500 µl blood loss (300 µg of iron), and a week later phlebotomized and placed on a 4 ppm iron-deficient diet. It is expected that the lack of hepcidin suppression in *Erfe* KO mice will impair compensatory increase in both the release of stored iron and the absorption of iron from the diet. Other outcomes would raise the possibility of differential regulation of intestinal versus macrophage iron transport as reported by others (Chaston 2008) or redundant pathways for their regulation.

Other possible activity: It is possible that Erfe exerts some influence on the marrow environment. To detect major effects one can compare the morphology of femur sections in Erfe-overexpressing, *Erfe^{-l-}* and WT mice. For comparison of the erythroid lineage in these mice, one can analyze the total spleen and bone marrow cells by flow cytometry based on the expression levels of c-kit, Ter119, CD71 and/or CD44. See Socolovsky 2007; Chen 2009.

### 2. Erythroferrone characterization, its activity and mechanism of action

The mass and composition of erythroferrone, including its intracellular and extracellular isoforms, and perform domain deletion analysis to establish its functional components may be characterized. Whether erythroferrone is secreted into the circulation and whether it exerts its effect directly on the liver may also be determined. Recombinant and/or synthetic forms of erythroferrone can be used to examine its effect on hepcidin expression *in vivo* and *in vitro.* The impact erythroferrone has on erythroid precursors and their differentiation can be examined as well as the receptor for erythroferrone.

Physicochemical and compositional characteristics - One can perform a medium scale transient transfection of HEK293T cells (Freestyle 293 Expression System, Invitrogen) with a plasmid encoding mouse and human erythroferrone cDNA sequence fused to a C-terminal FLAG and 6-Histidine tag. Expression in mammalian cells increases the likelihood that the recombinant protein will be as close to the native form as practically feasible. The intracellular form from cell lysates and the secreted form from the media may be purified and assayed. Based on prior experience, 2-step purification is preferred for obtaining a satisfactorily pure protein that can be used for *in vivo* and *in vitro* treatments. Therefore, the initial purification of recombinant erythroferrone can be done primarily using affinity beads specific for one of the C-terminal tags and final purification can be done by HPLC. To guard against potential inhibition of activity during purification or by tags, one may also test the activity of partially purified fractions and of protein treated to remove tags. The purified protein can be characterized as to the size using gel permeation chromatography and SDS-PAGE, and then the sequence can be determined by N-terminal Edman degradation and by endoprotease cleavage and fragmentation mass spectrometry. Using Western blots with antibodies against erythroferrone, one can compare the size of the recombinant protein to Erfe secreted by EPO-stimulated murine erythroblasts in culture.

*ERFE polypeptides and activity* - One can analyze the functional forms and domains of ERFE polypeptides by two complementary approaches. In the first, soluble and cell-derived ERFE partially or completely purified are tested for bioactivity by injection into mice. In the second approach, lentiviral delivery methods are used to study Erfe variants in which the protein is modified to remove parts or whole of its individual structural domains. Initially, one can substitute a generic signal sequence and subject the protein to N-terminal truncation analysis to remove the N-terminal domain (NTD1) and/or the collagen domain (Figure 12) to determine if these are critical for Erfe activity *in vivo.* N-terminal domain 2 (NTD2) may be more difficult to remove completely because its Cys142 is likely to participate in disulfide pairing but at least its N-terminal half will be removed. Based on its homology to other cytokines, it is expected that the TNF-like C-terminus is the active moiety and that the other domains facilitate synthesis, secretion or a possible alternative function such as cell-to-cell signaling of the membrane-associated form of Erfe. In both approaches, the readout 4-15 hours later will include serum hepcidin by an immunoassay and qPCR measurements of hepcidin mRNA expression in the liver. To prevent interference from the strong iron signal that stimulates hepcidin when mice are on "standard" diet (typically 270-300 ppm) which causes mild iron overload, mice can be placed on low (4 ppm Fe) or sufficient (50 ppm Fe) iron diet for 2 weeks prior to the injection. This preconditioning will assure responsiveness of hepcidin to both positive and negative stimuli.

*Erythroid maturation* - Erythroferrone could also exert effects on erythroid expansion and maturation, either directly or through its effects on iron delivery. Iron repletion after iron deficiency anemia was shown to increase nucleated erythroid cells 2.5-fold, and erythroblasts up to 3.9 fold, indicating that iron availability limits erythroblast expansion. In preliminary studies, flow cytometry (Ter119, CD71, CD44 markers) (Socolovsky 2007; Chen 2009) was used to analyze erythroblast maturation in the bone marrow and the spleen of phlebotomized wild-type, heterozygotes and KO mice compared to non-treated littermates controls. 72 hours after phlebotomy, erythroid cells in *Erfe*^{*-*/*-*} mice appeared to be less mature than in WT and heterozygotes, but more dead cells were not observed, thereby suggesting that Erfe does not play a role in erythroblast protection against apoptosis. Because the KO mice seem to have relatively fewer immature precursors (early erythroblasts) and apoptosis did not seem to be altered in *Erfe* KO mice, it is possible that Erfe could play a role in the regulation of early progenitor proliferation and differentiation.

One can study the role of erythroferrone in erythropoiesis by flow cytometry of splenic and marrow progenitors collected at various intervals after erythropoietic challenge as well cultured fetal liver progenitors, by comparing *Erfe* KO mice to WT mice. One can also study the effects of injected erythroferrone on erythropoiesis using similar methods in both *Erfe* KO and WT mice. Erythroblast differentiation can be characterized by Ter119, CD71 and/or CD44 expression. Apoptosis signaling and cell death can be assessed by staining with AnnexinV and 7-amino-actinomycin D (7-AAD), respectively.

The *in vivo* Erfe treatments followed by the flow cytometric analysis of erythroid precursor numbers and distribution between maturational stages will demonstrate any significant effects of Erfe on erythroid precursor proliferation or maturation. However, they will not establish whether the effects are mediated by iron and hepcidin or by the direct effect of Erfe on erythroid precursors. To answer this question, one can study the effect of Erfe added to fetal liver erythroid precursor cultures where iron (holotransferrin) is present in the medium and hepcidin concentrations are very low. *In vitro* culture of 14.5-15.5 day fetal liver erythroid precursors from WT and *Erfe^{-l-}* mice can be performed in the presence or absence of added recombinant Erfe. The maturation of erythroid precursors can be analyzed by flow cytometry as before. In all these studies, one can compare precursor populations or cell death/apoptosis indices between *Erfe* KO and WT mice and between Erfe- and sham-treated conditions.

*Receptors for ERFE* - Based on the similarity of erythroferrone to TNFα, the erythroferrone receptor may belong to the TNF receptor (TNFR) family. Several receptors in this family are orphans, i.e., lack any known ligands. See Bossen 2006. Thus, one can screen the expression library of known murine and human TNFR family by flow cytometry. The receptor constructs can be transiently expressed in HEK293 cells (Bossen 2006) and these can be stained with the FLAG-tagged form of murine or human erythroferrone (Figure 10G) followed by biotinylated anti-FLAG M2 antibody and PE-coupled streptavidin. If none of the known TNFR family bind erythroferrone, one can perform a robotic high throughput screen of all receptor-like molecules from a mouse and human cDNA expression library using adherent cells in 384-well plates and high throughput fluorescent microscopy. Hits can be subjected to flow cytometric confirmation. The expression pattern of all identified receptors can be screened by examination of tissue expression databases (e.g., Nextbio Body Atlas) to confirm that the receptor is expressed in the target tissue of erythroferrone (hepatocytes if the mediator acts directly), and to identify potential additional tissue and organ targets for erythroferrone. One can also undertake a biochemical approach to identify proteins interacting with erythroferrone. Erythroferrone and/or ERFE polypeptides can be immobilized on magnetic beads and used to extract any binding proteins from membrane or cell lysates of mouse primary hepatocytes (or other cell types identified as ERFE targets in preceding studies). The complex can be identified using HPLC-mass spectrometry. Once the erythroferrone receptor is identified, one can use its similarity to other receptors (if any) to pinpoint its potential signaling mechanisms.

### 3. Whether erythroferrone is increased in iron overload disorders associated with hepcidin suppression

Erythroferrone concentrations in healthy volunteers and patient serum samples as well as in mouse models may be assayed to confirm that erythroferrone acts as a pathological hepcidin suppressor in anemias with ineffective erythropoeisis.

*Normal and pathological erythroferrone concentrations* - Anti-erythroferrone antibodies directed against erythroferrone and/or ERFE polypeptides may be used to determine normal and abnormal erythroferrone concentrations in subjects. Anti-mouse Erfe antibodies against two different epitopes of the Erfe protein were found to react strongly with recombinant protein in Western blots (Figure 9C). ELISA assays may be used to measure circulating levels of ERFE in subjects including normal subjects and subjects after phlebotomy, hemolytic anemia or during recovery from anemia, and subjects suffering from iron disorders, including iron deficiency anemia and various forms of thalassemia, including thalassemia intermedia.

*Ablation of Erythroferrone* - Hepcidin is deficient in patients with thalassemia intermedia (Papanikolaou 2005; Origa 2007) and this explains why these patients hyperabsorb dietary iron and develop iron overload even without erythrocyte transfusions or any other source of nondietary iron. To explore the role of erythroferrone as a pathogenic hepcidin suppressor in β-thalassemia, one can analyze the effects of *Erfe* ablation on hepcidin expression and hepatic iron overload in a mouse model for thalassemia intermedia, such as Hbb^{th3/+}. The Hbb^{th3/+} mouse is the more severe of the two available models of β-thalassemia intermedia, with Hb levels 7-9 g/dL, 5-to 10-times increased circulating EPO concentrations and ineffective erythropoiesis including extramedullary sites. It exhibits low hepcidin but this eventually rises to "normal" levels as hepatic iron overload increases to 3- to 5-times that of strain controls by 6 months of age. See Gardenghi 2007; Nai 2012.

For example, Erfe levels were measured in 6 month-old Hbb^{th3/+} mice and their WT strain controls to determine whether high levels of Erfe could be responsible for the inappropriately low levels of hepcidin. It was shown that *Erfe* mRNA was greatly increased in the bone marrow and spleen of Hbb^{th3/+} mice compared to WT mice (Figure 11). To test whether increased Erfe is responsible for the iron overload observed in this model, one can ascertain the effect of ablating *Erfe* expression. The *Erfe* gene is on mouse chromosome 1 and the beta globin gene on mouse chromosome 7 and one can therefore generate Hbb^{th3/+} *Erfe*^{*-*/*-*} mice by breeding as was done for other genes in the thalassemia intermedia mice. See Nai 2012; Gardenghi 2010. The Hbb^{th3/+} mice on C57B/6J background can be obtained from Jackson Labs (B6.129P2-Hbb-b1tm1Unc Hbb-b2tm1Unc/J, stock number, 002683).

### References:

Ashby, D.R., et al. (2010). Erythropoietin administration in humans causes a marked and prolonged reduction in circulating hepcidin. Haematologica 95, 505-508.
Bossen, C., et al. (2006). Interactions of Tumor Necrosis Factor (TNF) and TNF Receptor Family Members in the Mouse and Human. J. Biol. Chem. 281, 13964-13971.
Bouscary, D., et al. (2003). Critical role for PI 3-kinase in the control of erythropoietin-induced erythroid progenitor proliferation. Blood 101, 3436-3443.
Bramey, T., et al. (2009). No evidence for protective erythropoietin alpha signalling in rat hepatocytes. BMC Gastroenterology 9, 26.
Casanovas, et al. (2013) The murine growth differentiation factor 15 is not essential for systemic iron homeostasis in phlebotomized mice. Haematologica. 98(3) :444-447.
Centis, F., et al. (2000). The importance of erythroid expansion in determining the extent of apoptosis in erythroid precursors in patients with β-thalassemia major. Blood 96, 3624-3629.
Chaston, T., et al. (2008). Evidence for differential effects of hepcidin in macrophages and intestinal epithelial cells. Gut 57, 374-382.
Chen, K., et al. (2009). Resolving the distinct stages in erythroid differentiation based on dynamic changes in membrane protein expression during erythropoiesis. PNAS USA 106, 17413-17418.
Clark, R.J., et al. (2011). Understanding the structure/activity relationships of the iron regulatory peptide hepcidin. Chem. Biol 18, 336-343.
Duran-Struuck and Dysko (2009). Principles of bone marrow transplantation (BMT): providing optimal veterinary and husbandry care to irradiated mice in BMT studies. J Am. Assoc. Lab. Anim Sci 48, 11-22.
England, S.J., et al. (2011). Immature erythroblasts with extensive ex vivo self-renewal capacity emerge from the early mammalian fetus. Blood 117, 2708-2717.
Fernandes, A., et al. (2009). The molecular basis of hepcidin-resistant hereditary hemochromatosis. Blood 114, 437-443.
Finch, C. (1994). Regulators of iron balance in humans. Blood 84, 1697-1702.
Ganz and Nemeth (2011). Hepcidin and disorders of iron metabolism. Annu. Rev. Med. 62, 347-360.
Ganz and Nemeth (2012). Iron metabolism: interactions with normal and disordered erythropoiesis. Cold Spring. Harb. Perspect. Med. 2, a011668.
Ganz, T., et al. Mini-Hepcidin Peptides and Methods of Using thereof. Patent Application. US 2012/0040894 A1. 6-10-2010.
Ganz, T., et al. (2008). Immunoassay for human serum hepcidin. Blood 112, 4292-4297.
Gardenghi, S., et al. (2010). Hepcidin as a therapeutic tool to limit iron overload and improve anemia in beta-thalassemic mice. J Clin. Invest 120, 4466-4477.
Gardenghi, S., et al. (2007). Ineffective erythropoiesis in β-thalassemia is characterized by increased iron absorption mediated by down-regulation of hepcidin and up-regulation of ferroportin. Blood 109, 5027-5035.
Ghoti, H., et al. (2011). Increased serum hepcidin levels during treatment with deferasirox in iron-overloaded patients with myelodysplastic syndrome. Br. J Haematol. 153, 118-120.
Goodnough, J.B., et al. (2012). Inhibition of hepcidin transcription by growth factors. Hepatology 56, 291-299.
Greene, W.K., et al. (2002). Enforced expression of HOX11 is associated with an immature phenotype in J2E erythroid cells. Br. J. Haematol 118, 909-917.
Horiuchi, T., et al. (2010). Transmembrane TNF-α: structure, function and interaction with anti-TNF agents. Rheumatology 49, 1215-1228.
Hrinda and Goldwasser (1969). On the mechanism of erythropoietin-induced differentiation. VI. Induced accumulation of iron by marrow cells. Biochim. Biophys. Acta 195, 165-175.
Huang, H., et al. (2009). Contribution of STAT3 and SMAD4 pathways to the regulation of hepcidin by opposing stimuli. Blood. 113(15): 3593-3599.
Karur, V.G., et al. (2006). Lyn kinase promotes erythroblast expansion and late-stage development. Blood 108, 1524-1532.
Kattamis, A., et al. (2006). The effects of erythropoetic activity and iron burden on hepcidin expression in patients with thalassemia major. Haematologica 91, 809-812.
Kimura, H., et al. (1986). Hematopoiesis in the rat: Quantitation of hematopoietic progenitors and the response to iron deficiency anemia. J. Cell. Physiol. 126, 298-306.
Lim, S.K., et al. (1998). Increased susceptibility in Hp knockout mice during acute hemolysis. Blood 92, 1870-1877.
Liu, Y., et al. (2006). Suppression of Fas-FasL coexpression by erythropoietin mediates erythroblast expansion during the erythropoietic stress response in vivo. Blood 108, 123-133.
Maes, K., et al. (2010). In anemia of multiple myeloma, hepcidin is induced by increased bone morphogenetic protein 2. Blood 116, 3635-3644.
Mastrogiannaki, M., et al. (2011). Hepatic HIF-2 down-regulates hepcidin expression in mice through epo-mediated increase in erythropoiesis. Haematologica.
Menon, M.P., et al. (2006). Signals for stress erythropoiesis are integrated via an erythropoietin receptor-phosphotyrosine-343-Stat5 axis. J Clin Invest 116, 683-694.
Merryweather-Clarke, A.T., et al. (2011). Global gene expression analysis of human erythroid progenitors. Blood 117, e96-e108.
Nai, A., et al. (2012). Deletion of TMPRSS6 attenuates the phenotype in a mouse model of beta-thalassemia. Blood 119, 5021-5029.
Nemeth, E., et al. (2006). The N-terminus of hepcidin is essential for its interaction with ferroportin: structure-function study. Blood 107, 328-333.
Nemeth, E., et al. (2004a). IL-6 mediates hypoferremia of inflammation by inducing the synthesis of the iron regulatory hormone hepcidin. J Clin. Invest 113, 1271-1276.
Nemeth, E., et al. (2005). Hepcidin is decreased in TFR2 hemochromatosis. Blood 105, 1803-1806.
Nemeth, E., et al. (2004b). Hepcidin regulates cellular iron efflux by binding to ferroportin and inducing its internalization. Science 306, 2090-2093.
Nemeth, E., et al. (2003). Hepcidin, a putative mediator of anemia of inflammation, is a type II acute-phase protein. Blood 101, 2461-2463.
Nicolas, G., et al. (2002). The gene encoding the iron regulatory peptide hepcidin is regulated by anemia, hypoxia, and inflammation. J Clin. Invest 110, 1037-1044.
Origa, R., et al. (2007). Liver iron concentrations and urinary hepcidin in beta-thalassemia. Haematologica 92, 583-588.
Pak, M., et al. (2006). Suppression of hepcidin during anemia requires erythropoietic activity. Blood 108, 3730-3735.
Papanikolaou, G., et al. (2005). Hepcidin in iron overload disorders. Blood 105, 4103-4105.
Park, C.H., et al. (2001). Hepcidin, a urinary antimicrobial peptide synthesized in the liver. J. Biol. Chem. 276, 7806-7810.
Pinto, J.P., et al. (2008). Erythropoietin mediates hepcidin expression in hepatocytes through EPOR signaling and regulation of C/EBPalpha. Blood 111, 5727-5733.
Pippard, M.J., et al. (1979). IRON ABSORPTION AND LOADING IN [beta]-THALASSEMIA INTERMEDIA. The Lancet 314, 819-821.
Porpiglia, E., et al. (2012). Stat5 Signaling Specifies Basal versus Stress Erythropoietic Responses through Distinct Binary and Graded Dynamic Modalities. PLoS Biol 10, e1001383.
Preza, G.C., et al. (2011). Minihepcidins are rationally designed small peptides that mimic hepcidin activity in mice and may be useful for the treatment of iron overload. J Clin. Invest 121, 4880-4888.
Qiao, B., et al. (2012). Hepcidin-induced endocytosis of ferroportin is dependent on ferroportin ubiquitination. Cell Metab 15, 918-924.
Ramos, E., et al. (2011). Evidence for distinct pathways of hepcidin regulation by acute and chronic iron loading in mice. Hepatology 53, 1333-1341.
Ramos, E., et al. (2012). Minihepcidins prevent iron overload in a hepcidin-deficient mouse model of severe hemochromatosis. Blood. 120(18): 3829-3836.
Ramos, P., et al. (2010). Iron metabolism and ineffective erythropoiesis in beta-thalassemia mouse models. Ann. N. Y. Acad. Sci. 1202, 24-30.
Ravasi, G., et al. (2012). Hepcidin expression in iron overload diseases is variably modulated by circulating factors. PLoS. ONE. 7, e36425.
Richmond, Terri D., et al. Turning cells red: signal transduction mediated by erythropoietin. Trends in Cell Biology 15[3], 146-155. 3-1-2005.
Rivera, S., et al. (2005a). Hepcidin excess induces the sequestration of iron and exacerbates tumor-associated anemia. Blood 105, 1797-1802.
Rivera, S., et al. (2005b). Synthetic hepcidin causes rapid dose-dependent hypoferremia and is concentrated in ferroportin-containing organs. Blood 106, 2196-2199.
Sadahira, Y., et al. (2000). Impaired splenic erythropoiesis in phlebotomized mice injected with CL2MDP-liposome: an experimental model for studying the role of stromal macrophages in erythropoiesis. J Leukoc Biol 68, 464-470.
Seldin, M.M., et al. (2012). Myonectin (CTRP15), a novel myokine that links skeletal muscle to systemic lipid homeostasis. J. Biol. Chem. 287, 11968-11980.
Sham, R.L., et al. (2009). Hereditary hemochromatosis due to resistance to hepcidin: high hepcidin concentrations in a family with C326S ferroportin mutation. Blood 114, 493-494.
Socolovsky, M. (2007). Molecular insights into stress erythropoiesis. Curr. Opin. Hematol. 14, 215-224.
Tanno, T., et al. (2007). High levels of GDF15 in thalassemia suppress expression of the iron regulatory protein hepcidin. Nat. Med. 13, 1096-1101.
Tanno, T., et al. (2010a). Growth differentiation factor 15 in erythroid health and disease. Curr. Opin. Hematol. 17, 184-190.
Tanno, T., et al. (2009). Identification of TWSG1 as a second novel erythroid regulator of hepcidin expression in murine and human cells. Blood 114, 181-186.
Tanno, T., et al. (2010b). Expression of growth differentiation factor 15 is not elevated in individuals with iron deficiency secondary to volunteer blood donation. Transfusion 50, 1532-1535.
Teglund, S., et al. (1998). Stat5a and Stat5b proteins have essential and nonessential, or redundant, roles in cytokine responses. Cell 93, 841-850.
Vokurka, M., et al. (2006). Hepcidin mRNA levels in mouse liver respond to inhibition of erythropoiesis. Physiol Res. 55, 667-674.
Wong, S., et al. (2010). Establishment of an erythroid cell line from primary CD36+ erythroid progenitor cells. Exp. Hematol. 38, 994-1005.
Zhang, J., et al. (2003). Role of Ras signaling in erythroid differentiation of mouse fetal liver cells: functional analysis by a flow cytometry-based novel culture system. Blood 102, 3938-3946.

Having thus described exemplary embodiments of the present invention, it should be noted by those skilled in the art that the within disclosures are exemplary only and that various other alternatives, adaptations, and modifications may be made within the scope of the present invention. Accordingly, the present invention is not limited to the specific embodiments as illustrated herein, but is only limited by the following claims.

## Claims

1. A monoclonal antibody which specifically recognizes an ERFE polypeptide or a composition thereof for use in treating an iron overload disease and/or disorder associated with abnormally low levels of hepcidin, wherein the ERFE polypeptide comprises an amino acid sequence selected from at least one of SEQ ID Nos: 1, 3, 4, 6, 7, 9, 10, 12-14, AHSVDPRDAWMLFVX₁QSDKGX₂N (SEQ ID NO:5) wherein X₁ is R and X₂, is V; VX₁RRALHELGX₂YYLPX₃ (SEQ ID NO:8) wherein X₁ is E, X₂, is V and X₃, is D; X₁MGLE X₂SSELFTISVNGVLYLQ (SEQ ID NO:11) wherein X₁ is I and X₂, is S; and SLTVRSGSHFSAX₁LLGX₂ (SEQ ID NO:15) wherein X₁ is V and X₂, is V, wherein the iron overload disease and/or disorder associated with abnormally low levels of hepcidin is selected from the group consisting of: myelodysplastic syndromes, hereditary hemochromatosis, iron-loading anemias, alcoholic liver diseases, chronic hepatitis C, alpha-thalassemia, beta-thalassemia, congenital dyserythropoietic anemias, and chronic hepatitis B.

2. The monoclonal antibody or a composition thereof for the use of claim 1, wherein the composition comprises a pharmaceutically acceptable carrier or diluent.

3. The monoclonal antibody or a composition thereof for the use of claim 1 or claim 2, wherein the monoclonal antibody is raised against a polypeptide comprising an amino acid sequence of SEQ ID NO: 1.

## Patentansprüche

1. Ein monoklonaler Antikörper, der spezifisch ein ERFE-Polypeptid erkennt, oder eine Zusammensetzung davon, zur Verwendung bei der Behandlung einer Eisenspeicherkrankheit und/oder einer Störung, die mit abnormal niedrigen Spiegeln von Hepcidin assoziiert ist, wobei das ERFE-Polypeptid eine Aminosäuresequenz umfasst, die ausgewählt ist aus mindestens einer der SEQ ID Nos: 1, 3, 4, 6, 7, 9, 10, 12-14, AHSVDPRDAWMLFVX₁QSDKGX₂N (SEQ ID NO:5), wobei X₁ R ist und X₂ V ist; VX₁RRALHELGX₂YYLPX₃ (SEQ ID NO:8), wobei X₁ E ist, X₂ V ist und X₃ D ist; X₁MGLEX₂SSELFTISVNGVLYLQ (SEQ ID NO: 11), wobei X₁ I ist und X₂ S ist; und SLTVRSGSHFSAX₁LLGX₂ (SEQ ID NO: 15), wobei X₁ V ist und X₂ V ist, wobei die Eisenspeicherkrankheit und/oder die Störung, die mit abnormal niedrigen Spiegeln von Hepcidin assoziiert ist, ausgewählt ist aus der Gruppe bestehend aus: myelodysplastischen Syndromen, hereditärer Hämochromatose, Eisenspeicher-Anämie, alkoholischen Leberkrankheiten, chronischer Hepatitis C, Alpha-Thalassämie, Beta-Thalassämie, kongenitaler dyserythropoetischer Anämie und chronischer Hepatitis B.

2. Der monoklonale Antikörper oder eine Zusammensetzung davon zur Verwendung von Anspruch 1, wobei die Zusammensetzung einen pharmazeutisch akzeptablen Träger oder Verdünner umfasst.

3. Der monoklonale Antikörper oder eine Zusammensetzung davon zur Verwendung von Anspruch 1 oder Anspruch 2, wobei der monoklonale Antikörper gegen ein Polypeptid erzeugt wurde, das eine Aminosäuresequenz von SEQ ID NO:1 umfasst.

## Revendications

1. Anticorps monoclonal qui reconnaît spécifiquement un polypeptide ERFE ou une composition de celui-ci pour une utilisation dans le traitement d'une maladie et/ou d'un trouble de surcharge en fer associé(e) à des taux anormalement bas d'hepcidine, dans lequel le polypeptide ERFE comprend une séquence d'acides aminés choisie parmi au moins l'une de SEQ ID Nos: 1, 3, 4, 6, 7, 9, 10, 12-14, AHSVDPRDAWMLFVX₁QSDKGX₂N (SEQ ID NO:5) où X₁ représente R et X₂ représente V ; VX₁RRALHELGX₂YYLPX₃ (SEQ ID NO:8) où X₁ représente E, X₂ représente V et X₃ représente D ; X₁MGLEX₂SSELFTISVNGVLYLQ (SEQ ID NO:11) où X₁ représente I et X₂ représente S ; et SLTVRSGSHFSAX₁LLGX₂ (SEQ ID NO:15) où X₁ représente V et X₂ représente V, la maladie et/ou le trouble de surcharge en fer associé(e) à des taux anormalement bas d'hepcidine étant choisi(e) dans le groupe consistant en les syndromes myélodysplasiques, l'hémochromatose héréditaire, les anémies de charge en fer, les maladies hépatiques alcooliques, l'hépatite C chronique, l'alpha-thalassémie, la bêta-thalassémie, les anémies dysérythropoïétiques congénitales et l'hépatite B chronique.

2. Anticorps monoclonal ou composition de celui-ci pour l'utilisation selon la revendication 1, la composition comprenant un support ou diluant pharmaceutiquement acceptable.

3. Anticorps monoclonal ou composition de celui-ci pour l'utilisation selon la revendication 1 ou la revendication 2, l'anticorps monoclonal étant dirigé contre un polypeptide comprenant une séquence d'acides aminés de SEQ ID NO:1.
